# EUROPEAN PATENT APPLICATION

(11) **EP 4 520 338 A1**
(43) Date of publication of application: **12.03.2025**
(21) Application number: 23799756.4
(22) Date of filing: 11.04.2023
(51) Int. Cl.: A61K 33/04, A61K 33/06, A61K 35/64, A61K 36/28, A61K 36/64, A61P 11/00

(54) **HOMEOPATHIC PHARMACEUTICAL COMPOSITION FOR TREATING UPPER AND LOWER RESPIRATORY TRACT DISEASES**

(30) Priority: 06.05.2022 RU 2022112392
(71) Applicant: Nersesyan, Marina Vladislavovna, Moscow, 125414 (RU)
(72) Inventor: Nersesyan, Marina Vladislavovna, Moscow, 125414 (RU)
(74) Representative: Kuttenkeuler, David
(86) International application number: PCT/RU2023/050082
(87) International publication number: WO 2023/214899

(57) **Abstract**

The invention relates to a pharmaceutical composition for treating or preventing acute and chronic inflammatory, allergic, post-traumatic, atrophic and hypertrophic diseases of the upper and lower respiratory tracts, and to the use thereof. The pharmaceutical composition comprises alcoholic tinctures of *Tartarus stibiatus, Hepar sulfur, Euphrasia officinalis, Allium cepa* and *Arnica montana* and an alcoholic solution of *Apis mellifica,* which are mixed with a physiologically acceptable carrier.

## Description

The present inventions relate to homeopathic medicine and pharmaceuticals and include pharmaceutical compositions based on natural herbal components intended for treating or preventing acute and exacerbated chronic inflammatory and allergic respiratory diseases, which also have an anti-inflammatory, anti-edematous, mucolytic, wound-healing, restorative (in relation to the respiratory tract epithelium) effect and accelerate recovery from acute viral and bacterial infections of the upper and lower respiratory tract, the pharmaceutical compositions comprising the effective amounts of alcoholic tinctures of herbal components, including *Euphrasia officinalis, Allium cepa,* and *Arnica montana,* and, optionally, the effective amounts of alcoholic tinctures of the components of animal and mineral origin, including *Tartarus stibiatus, Hepar sulfur,* and an alcoholic solution of *Apis mellifica,* and to the use of the said homeopathic pharmaceutical composition for treating or preventing acute and exacerbated chronic inflammatory and allergic diseases of the upper and lower respiratory tract.

The composition of the present invention is free of non-natural components; it has a synergistic anti-inflammatory, anti-edematous, restorative, and mucociliary clearance improving effect in the said upper and lower respiratory tract diseases that allows for remission in chronic inflammatory and allergic respiratory diseases, in particular, in chronic inflammatory and allergic diseases of the nasal mucosa and paranasal sinuses and has restorative properties in relation to the ciliated epithelium of the upper and lower respiratory tract.

Due to the mutual potentiation of the components taken together, the proposed group of inventions provides for a number of technical results, including:
- a super-cumulative (synergistic) improvement of mucociliary clearance, a non-specific mechanism that provides local protection of the respiratory mucosa from external influences, in particular from exogenous pathogenic agents (bacteria, viruses, etc.) and allergens,
- a super-cumulative inhibition of upper and lower respiratory tract ciliated epithelial cytolysis upon inflammation,
- simplified therapy of acute and exacerbated chronic inflammatory and allergic diseases of the upper respiratory tract, as well as allergic and post-traumatic conditions of the nasal mucous membrane and paranasal sinuses,
- a super-cumulative restorative and anti-edematous effect, including in inflammatory, allergic, and post-traumatic conditions of the mucous membrane,
- a super-cumulative (synergistic) increase in the therapeutic activity of the pharmaceutical composition due to the combined use of natural herbal ingredients and, optionally, ingredients of animal and mineral origin in its formulation,
- simplified management, mitigation, or inhibition of inflammatory and allergic diseases of the upper and lower respiratory tract,
and expanded range of topical pharmaceutical compositions intended for treating and preventing acute and chronic inflammatory and allergic diseases of the upper and lower respiratory tract with anti-inflammatory, anti-edematous, mucolytic, and restorative effects.

### Prior Art

Inflammatory diseases of the upper respiratory tract (nasal cavity, paranasal sinuses, and nasopharynx) mucous membrane and especially inflammatory diseases of the nasal mucous membrane (rhinitis) and paranasal sinuses (sinusitis), as well as rhinosinusitis, in all the diversity of their manifestations, are among the most common diseases in healthcare today. Clinical manifestations and the severity of these disorders may vary; without treatment, these disorders may often become complicated. In these cases, treatment may require the involvement of multidisciplinary medical practitioners, such as ENT specialists, pulmonologists, allergists, neurologists, and even intensive care specialists [Fokkens WJ, Lund VJ et al., Rhinology 2012 Vol.50 Suppl. 23. No.3, pp. 1-298]*.*

Depending on its etiology, rhinosinusitis can be viral, bacterial, fungal, allergic, and mixed acute. The clinical symptoms in the first 5 days are similar, but the required treatment and outcome are fundamentally different [2-6].

Acute viral rhinosinusitis presents a special problem. The incidence of acute viral rhinosinusitis (ARVI) is very common; on average, 2 to 5 times a year in adults, and 7 to 10 times a year in school-age children [Van Gageldonk-Lafeber AB, Heijnen ML, Barteids Al, Peters MF, van der Plas SM, Wilbrink B., Clin Infect Dis. 2005 Aug 15; 41(4):490-7. Epub 2005 Jul 15], while, according to the American Rhinologic Society, about 2% of acute rhinosinusitis cases are complicated by bacterial infections *[*Cherry DK, Woodwell DA, Rechtsteiner EA., Adv Data. 2007 Jun 29(387): 1-39; Bhattacharyya N., Am J Rhinol Allergy 2009; 23(4):392-5*;* Bhutta MF, Al-Shaikh S, et al., Rhinology 2011 Jun; 49(2): 185-9]; however, their detection and treatment is often complicated, as many rhinosinusitis patients prefer self-treatment rather than seeking medical assistance.

A recent case-control study by Danish colleagues showed that 900,000 patients a year are diagnosed with acute respiratory viral infections, of which 24% are diagnosed with viral rhinosinusitis and 11% are diagnosed with influenza. In 18 out of 1,000 children aged 12 to 17 years old and in 2 out of 1,000 children aged 0-4 years old, ARVI is complicated by sinusitis. In 2004, *Uijen et al.* noted an incidence of 7/1,000 in 2002 and 4/1,000 by 2008 (p < 0.001) [Bachert C, Hormann K, Mosges R, Rasp G, Riechelmann H, Muller R, et al., Allergy. 2003 Mar; 58(3): 176-91*;* Uijen JH, Bindels PJ, Schellevis FG, van der Wouden JC., Scand J Prim Health Care. 2011 Jun; 29(2):75-9*].*

In Germany, from July 2000 to June 2001, 6.3 million people were diagnosed with acute respiratory viral infections, and 8.3 million people were diagnosed with acute rhinosinusitis [Bachert C, Hormann K, Mosges R, Rasp G, Riechelmann H, Muller R, et al. An update on the diagnosis and treatment of sinusitis and nasal polyposis. // Allergy. 2003 Mar; 58(3): 176-91].

According to the American Rhinologic Society, about 2% of acute rhinosinusitis cases are complicated by bacterial infections, but colleagues noted that the actual number of complications is difficult to estimate due to the low number of people seeking assistance [Cherry DK, Woodwell DA, Rechtsteiner EA., Adv Data. 2007 Jun 29(387): 1-39*;* Bhattacharyya N., Am J Rhinol Allergy 2009; 23(4):392-5*;* Bhutta MF, Al-Shaikh S, et al., Rhinology 2011 Jun; 49(2): 185-9]. The main symptoms of acute viral rhinosinusitis include nasal breathing difficulty or nasal congestion (nasal block), nasal discharge (runny nose) and drainage of secretions down the back of the throat, facial pain, pressure and distension in the sinus area, and a reduced smell sensitivity (hyposmia) or loss of smell (anosmia). According to the San Francisco Medical Center, acute sinusitis was diagnosed in 12% of patients from 1998 to 1999, or 1,601 out of 13,740 patients who were admitted for treatment *[*Louie JK, Hacker JK, Gonzales R, Mark J, Maselli JH, Yagi S, et al., Clinical infectious diseases: an official publication of the Infectious Diseases Society of America. 2005 Sep 15; 41(6):822-8*,* Varonen H, Rautakorpi UM, Huikko S, Honkanen PO, Klaukka T, Laippala P, et al., Scand J Prim Health Care. 2004 Jun; 22(2): 122-7*]. Rautakorpi et al.* reported that, in Asia, 12% of patients admitted for consultation due to acute respiratory viral infections had acute rhinosinusitis [Wang DY, Wardani RS, Singh K, Thanaviratananich S, Vicente G, Xu G, et al., Rhinology 2011 Sep; 49(3):264-71*].*

The symptoms of acute bacterial rhinosinusitis, which can be caused by various pathogens, such as *Streptococcus pneumoniae, Haemophilus influenza, Moraxella catarrhalis, anaerobic bacteria, Staphylococcus aureus,* etc., are usually less pronounced, or smoothed; the outcome in such cases of rhinosinusitis may be self-recovery. However, a well-known Cochrane study showed the need to use antibacterials in acute bacterial rhinosinusitis. Therefore, in the USA, according to the National Ambulatory Medical Care Survey (NAMCS), sinusitis is one of the top five diagnoses for which antibacterial therapy is most often prescribed. Retrospectively, in 2002, antibacterial therapy was prescribed to 9% to 21% of pediatric and adult patients [Gijsen R., Poos M. National Kompas 2003 *[cited; Available from:* Gwaltney JM, Jr Acute community-acquired sinusitis.-Clin Infect Dis 1996; 23(6): 1209-23*; quiz.24-5].*

In addition, the treatment of acute bacterial rhinosinusitis with the commercially available antibacterial agents may be challenged due to the widespread prevalence of strains of these pathogens resistant to the antibiotics used [Felmingham D., Feldman C. et al., Clin. Microbiol. Infect 2002; 8 Suppl. 2: 12-42*,* Hoban D., Felmingham D., J. Antimicrob. Chemother. 2002 Sep.; 50 Suppl. SI: 49-59*].*

Bacteriological tests take a long time (from 5 to 7 days); therefore, antibiotics are often prescribed empirically, whereas when choosing antibacterial therapy, the medical practitioner should consider not only the potential resistance of many bacteria to the antibacterial agents used but also the potential generation of biofilms, which are associations of various bacteria with different sensitivities to various antibacterial agents. All this complicates the correct selection of an antibacterial agent for treating acute uncomplicated rhinosinusitis. Moreover, as experts are aware, the improper treatment of acute rhinosinusitis can cause the disease to become chronic.

### Allergy, Surgery, and Chronic Rhinosinusitis

Benninger [Benninger MS., Otolaryngol Head Neck Surg. 2008 Mar; 138(3):274-8] showed that almost 54% of outpatients with chronic rhinosinusitis (CRS) have concurrent allergies.

Allergy symptoms include a pronounced swelling of the mucous membrane, leading to obstruction or closure of the natural openings of the paranasal sinuses and decreased ventilation of the sinuses, which causes congestion of secretions in the sinuses; all these manifestations are predisposing factors for the development of rhinosinusitis and its further chronicity [Bachert C, Schapowal A, Funk P, Kieser M., Rhinology 2009 Mag; 47(1):51-8]. *Chen et al.* studied 8,723 children and found that the prevalence of rhinosinusitis was significantly higher in children with allergies than in those without them [Chen Y, Dales R, Lin M., The Laryngoscope 2003 Jul; 113(7): 1199-205*].*

According to Benninger, among patients undergoing endoscopic sanitation of the paranasal sinuses in CRS, an allergy was detected in 50% to 84% of patients, and it is polyvalent in most of them [*Benninger MS., 2008*]*.* According to Friedman, 94% of patients with chronic rhinosinusitis who undergo sphenoethmoidectomy are diagnosed with allergies. Therefore, it can be concluded that an allergy may trigger the development of rhinosinusitis and its chronicity; in addition, in patients with allergies, upper respiratory tract infections are much more often complicated by rhinosinusitis [Friedman WH., Laryngoscope 1975 Dec; 85(12 pt 1): 1999-2011].

### Epidemiology of Rhinosinusitis in Children

There are a number of published prospective studies investigating the epidemiology of rhinosinusitis in children. For example, Maresh and Washburn studied 100 children from birth to 12 years old [Maresh MM, Wahbum A., Am J Dis Child. 1940; 60(4):841-861*]* and showed that about 30% of children under 6 years of age have chronic inflammatory diseases of the upper respiratory tract (rhinosinusitis, adenoiditis, etc.); by the age of 12, the percentage of chronic diseases decreases to 15%. In almost half of the cases of acute respiratory viral infections in children that last more than 2 weeks, the disease is complicated by sinusitis. Moreover, according to the authors, surgical treatment, in particular tonsillectomy, does not improve the findings. Clinically, rhinosinusitis in children is manifested by prolonged congestive, often purulent, nasal discharge and nasal congestion or difficulty breathing through the nose. Almost 80% of children with mucous edema detected by endoscopy have difficulty breathing through the nose or even nasal congestion, while 100% of such children are diagnosed with the same disorders when purulent discharge is visualized endoscopically [Bagatsch K DK, Paethenheimer F, Ritter B., HNO Praxis. 1980(5): 1-8].

Since the introduction of spiral computed tomography (SCT), sinusitis in children with the above complaints has been revealed in 64% of cases while, using magnetic resonance imaging (MRI) data, this figure is 45% [Gordts F, Clement PA, Destryker A, Desprechins B, Kaufman L., Rhinology 1997 Dec; 35(4): 154-7*,* van der Veken PJ, Clement PA, Buisseret T, Desprechins B, Kaufman L, Derde MP, Rhinology 1990 Sep; 28(3): 177-84].

Chronic rhinosinusitis (CRS) results in poor health outcomes for patients and significant financial costs to society, both directly related to the costs of treating patients with CRS and their reduced capacity to work. A recent study of the English-language literature showed that, in the United States, *Ray et al.* in their survey paper, using data from the U.S. National Center for Health Statistics, stated that the costs of the direct treatment of patients with CRS (medical and surgical) amount to about $5.76 billion [Ray NF, Baraniuk JN, Thamer M, Rinehart CS, Gergen PJ, Kaliner M, Josephs S, Pung YH., J Allergy Clin Immunol. 1999 Mar; 103(3 Pt I):408-14]. The cost of CRS patient consultation amounts to about $3.39 billion, not including the costs of X-ray examinations, pharmacotherapy, and the patient's reduced capacity to work. According to the United States Department of Health and Human Services, the total number of sick leave days due to rhinosinusitis was 12.5 million per year, while the number of days of decreased work capacity due to rhinosinusitis was 568.7 million days [Stankiewicz JA, Chow JM., Am J Rhinol. 2003 May-Jun; 17(3): 139-42*,* Franzese CB, Stringer SP., Am J Rhinol. 2004 Sep-Oct; 18(5):329-34]. According to *Murphy et al.,* CRS patients have 43% more outpatient consultations and 25% more emergency consultations compared to the general population (*p = 0.001*) [Murphy MP, Fishman P, Short SO, Sullivan SD, Yueh B, Weymuller EA Jr., Otolaryngol Head Neck Surg. 2002 Nov; 127(5):367-76]. According to them, the total cost of treating CRS patients is $2.609 billion per year [Blackwell DL1, Collins JG, Coles R., Vital Health Stat 10. 2002 May; (205): 1-109*,* Murphy MP1, Fishman P, Short SO, Sullivan SD, Yueh B, Weymuller EA Jr., Otolaryngol Head Neck Surg. 2002 Nov; 127(5):367-76].

Only one similar study has been conducted in Europe, which found that the annual cost of direct treatment of CRS patients in the Netherlands was $1,861 per year [Van Agthoven M, Uyl-de Groot CA, Fokkens WJ, van de Merwe JP, Busschbach JJ., Rhinology. 2002 Jun; 40(2):69-74*].*

However, the actual costs of CRS are much higher. 85% of CRS patients are of working age (18 to 65 years old); therefore, taking into account the number of days of reduced work capacity and missed work days due to illness, the costs of treating CRS will be significantly higher. A survey paper by *Goetzel et al.,* including 2003 published papers, showed that CRS is among the top 10 most costly conditions in the United States [Goetzel RZ, Hawkins K, Ozminkowski RJ, Wang S., J Occup Environ Med. 2003 Jan; 45(1):5-14*].*

According to Bhattachaya, the cost of treating CRS patients is $1,539 per year, while 32 million Americans suffer with CRS. Therefore, the total cost of treatment for the U.S. budget is about 47 billion for all patients with various types of rhinosinusitis *[*Bhattacharyya NI., Am. J Rhinol. 2003; 17(1):27-32*].*

According to the latest research by Gliklich and Metson, annual treatment costs in 1998 were approximately $1,220 per year, including: symptomatic treatment agents ($198), nasal sprays ($250), and antibiotics ($772) [Gliklich RE, Metson R., Otolaryngol Head Neck Surg. 1998 Mar; 118(3 Pt I):344-9].

In a survey paper by *Wasserfallen et al.* on the pharmacoeconomics of antibiotic use, the authors emphasize the importance of symptomatic treatment of acute rhinosinusitis. They believe that antibiotics should only be prescribed if patients do not improve after 7 days of symptomatic treatment and state that this is the most effective, cost-efficient way to treat acute rhinosinusitis [Wasserfallen JB1, Livio F, Zanetti G., Pharmacoeconomics. 2004;22(13):829-37*].*

According to Wikipedia, the number of acute rhinosinusitis cases in Russia is 750,374 per year, while the number of chronic rhinosinusitis cases is 1,863,757 per year.

All of the above confirms the suffering of patients with rhinosinusitis, the general worse health of patients with CRS, and their decreased quality of life compared to the rest of the population. The following groups of rhinosinusitis drugs are currently available on the Russian pharmaceutical market:
1). Solutions for irrigation (rinsing) of the nasal cavity, consisting of an isotonic or hypertonic solution of mineral salts (sea water), sometimes with the addition of antiseptics.
This group includes topical agents aimed at mechanically washing away drying secretions (crusts) from the mucous membrane and mechanical (along with the washing fluid) removal of viruses, bacteria, and dust particles inhaled with air and settling on the nasal mucous membrane that may also contain allergens. Another recommendation for the use of drugs of this group is constant hygiene of the nasal cavity and temporary moistening of the mucous membrane for 5 to 15 minutes, depending on the individual duration of the nasal cycle according to the saccharin test [Rikhelman G., Lopatin A.S., Russian Rhinology, 1994, No. 4, pp. 33-47]*.*

The following products from this group should be noted:

### a) seawater nasal rinse preparations:

**Marimer** (manufactured by Laboratories Gilbert, France): sterile isotonic seawater solution.

**Rhinorin** (manufactured by Orion Corporation, Finland): sodium chloride, potassium chloride, calcium chloride + excipients: benzalkonium chloride, sodium hydroxide, hydrochloric acid (to ensure pH and stabilization) and purified water.

**Aqualor** (manufactured by AQUALOR LLC (Russia), LABORATOIRES CHEMINEAU (France), YS LAB (France), AURENA LABORATORIES (Sweden)): natural sea water with NaCl isotonic concentration of 8 to 11 g/l and pH 6.0 to 8.5.

**Dolphin** (manufactured by Dolphin LLC, Russia): plant and mineral complex based on rock salt (halite) containing a large number of micronutrients, such as potassium, magnesium, calcium, sulfate, sodium, etc. (with the addition of biologically active substances of licorice root and rose hip, eucalyptus oil, menthol, camphor oil). **Physiomer** (manufactured by Laboratoires Goemar, France): sea water (0.9% solution).

**Aqua Maris** (manufactured by JADRAN-GALENSKI LABORATORIJ d.d., Croatia): sterilized water from the Adriatic Sea with natural micronutrients (Na, Ca, Mg, Cl, SO4, HNO3).

**Quixx** (manufactured by Berlin-Chemie/Menarini): Atlantic Ocean water (2.6% salt content).

**Aquamaster** (manufactured by EVALAR JSC, Russia): an isotonic, hypertonic solution.

**Salin** (manufactured by Sagmel Inc., USA): 0.65% isotonic aqueous solution of sodium chloride further containing benzalkonium chloride, sodium dihydrogen phosphate dihydrate, sodium hydrogen phosphate dihydrate, and purified water.

**Rhinorin** (manufactured by Orion Corporation, Finland): isotonic saline solution, contains sodium chloride, potassium chloride, calcium chloride, excipients such as benzalkonium chloride, sodium hydroxide, hydrochloric acid (to ensure pH and stabilization), and purified water.

All of the above products have a similar mechanism of action: mechanical cleansing of the nasal cavity and removing bacteria and viruses, as well as dust particles from the nasal mucous membrane. These products do not contain drugs that can pathogenetically or symptomatically affect inflammation or allergies. In addition, the available literature shows that regular rinsing (hygiene) of the nasal cavity without indications washes away the thin mucous layer covering the nasal mucous membrane, the mucous layer containing protective substances such as mucin, lysozyme

(mononuclear phagocytes) produced by goblet and cylindrical cells of the mucous membrane and having bacteriostatic properties due to their ability to break the glycosidic bonds of polymer N-glucosamines that are present in the cell walls of gram-positive bacteria. These protective substances are actually the first protective natural barrier (microbiota) for inhaled viruses and bacteria. Therefore, washing away this protective layer can lead to decreased body resistance to pathogenic bacteria and viruses [Fokkens WJ, Lund VJ et al. EPOS 2012 // Rhinology 2012 Vol.50 Suppl. 23. No. 3, p. 1-298*].*

### b) topical antibacterial agents and topical antiseptics.

**Bioparox** (manufactured by Laboratories Servier): contains the antibiotic fusafungine, the use of which is limited in sinusitis due to the very frequent cases of resistance to other antibacterial agents and to common side effects, such as bronchospasm and even anaphylactic shock; the product is currently out of production.

**Polydexa with phenylephrine** (manufactured by Laboratoires BOUCHARA-RECORDATI, France), ingredients: neomycin sulfate, polymyxin B sulfate, dexamethasone sodium metasulfobenzoate, phenylephrine hydrochloride, a complex corticosteroid agent with phenylephrine providing a vasoconstriction effect and two antibacterial agents (a combination of neomycin and polymyxin B); to be used only when prescribed by a medical practitioner since there is a high risk of developing resistance to antibacterial agents and due to the high risk of allergic and systemic reactions to the components of the treatment agent.

**2) The second group includes** the following mucolytics:
**Rinofluimucil** (manufactured by ZAMBON, S.p.A., Italy): Acetylcysteine + Tuaminoheptane Rinofluimucil^{®} provides mucolytic, anti-edematous effects. Acetylcysteine contained in the treatment agent has a thinning effect on mucous and purulent mucosal discharge by breaking the disulfide bonds of mucus glycoproteins. Acetylcysteine also has an anti-inflammatory effect (leukocyte chemotaxis inhibition) and demonstrates antioxidant properties. This treatment agent is contraindicated in children (under 3 years of age), patients with bronchial asthma, arterial hypertension, functional class III to IV angina pectoris, frequent extrasystole.

**Lasolvan Rhino** (manufactured by Istituto de Angeli S.R.L., Italy), ingredients: tramazoline hydrochloride monohydrate, excipients: citric acid monohydrate-270 µg; sodium hydroxide-154 µg; benzalkonium chloride-14 µg; hypromellose (hydroxypropyl methylcellulose)-35 µg; povidone-2,101 µg; glycerol 85%-700 µg; magnesium sulfate heptahydrate-49 µg; magnesium chloride hexahydrate-35 µg; calcium chloride dihydrate-11 µg; sodium hydrogen carbonate-1 µg; sodium chloride-183 µg; cineole (eucalyptol)-7 µg, L-menthol (levomenthol)-14 µg; racemic camphor-14 µg; purified water; tramazoline hydrochloride is a decongestant active ingredient of Lasolvan^{®} Rhino and an α₂-adrenomimetic, which causes vasoconstriction; when applied to the nasal mucous membranes, the treatment agent reduces swelling due to its vasoconstrictive effect. As a result, the patency of the nasal passages is quickly restored, and nasal breathing is facilitated for a long time. It is known that long-term use of nasal vasoconstrictors can lead to the development of chronic inflammation and nasal congestion, as well as to nasal mucosa atrophy.

**3) The third group includes antihistamine agents** (prescribed for allergies). According to the latest recommendations of the American Rhinologic Society and the European Rhinologic Society, antihistamines are not recommended for use in acute inflammatory and exacerbated chronic rhinitis and rhinosinusitis, which significantly narrows the indications for their use:
**Cetrine,** cetirizine dihydrochloride tablets: a histamine H₁-receptor blocker; indicated for seasonal and year-round allergic rhinitis; allergic conjunctivitis; pollinosis (hay fever); urticaria (including chronic idiopathic); other allergic dermatosis manifestations (including atopic dermatitis and neurodermatitis) accompanied by itching and rashes; angioedema (Quincke's edema).

**Aerius,** desloratadine (micronized): a long-acting antihistamine agent, a blocker of peripheral histamine H₁-receptors; desloratadine is the primary active metabolite of loratadine and prevents the development and relieves the course of allergic reactions; it has antipruritic and antiexudative effects, reduces capillary permeability, prevents tissue edema development and smooth muscle spasm. This treatment agent is indicated for allergic rhinitis (to eliminate or relieve sneezing, nasal congestion, nasal discharge, itching in the nose, itching of the palate, itching and redness of the eyes, lacrimation), urticaria (to reduce or eliminate skin itching, rash); it is excreted by the kidneys and liver; allergic reactions are possible.

A side effect of antihistamines is a significant thickening of nasal discharge and a disturbance of its evacuation from the nasal cavity and paranasal sinuses.

### 5) Herbal treatment agents

**Pinosol** (manufactured by ZENTIVA a.s., Slovakia): nasal drops containing Scots pine oil, eucalyptus oil, thymol, tocopherol acetate, peppermint oil, guaiazulene, excipients: butylhydroxyanisole, macrogol esters, and apricot oil glycerides (Labrafil M-1944-CS), vegetable oil.

Pinosol is a herbal decongestant. It provides anti-inflammatory and antiseptic effects and is indicated in acute and chronic rhinitis, atrophic rhinitis, acute and chronic inflammatory diseases of the nasal and nasopharyngeal mucous membranes accompanied by dryness of the nasal mucous membranes. It is also prescribed in the postoperative period after surgery in the nasal cavity under the supervision of a medical practitioner. Local reactions are possible: itching, burning sensation, hyperemia, and swelling of the nasal mucosa. Pinosol is also contraindicated in allergies, including allergic rhinitis.

**Sinupret** (manufactured by Bionorica CE, Germany): contains gentian (Gentiana lutea) root, primrose (Primula veris) flowers, sorrel (Rumex acetosa) herb, elder (Sambucus nigra) flowers, verbena (Verbena officinalis) herb; excipients: potato starch, colloidal silicon dioxide, purified water, lactose monohydrate, gelatin, sorbitol, stearic acid.

Sinupret provides a secretolytic, secretomotor, anti-inflammatory, anti-edematous, moderate antibacterial, and antiviral effect and promotes the outflow of exudate from the paranasal sinuses and upper respiratory tract. However, the use of Sinupret for acute and chronic sinusitis is limited, as it is accompanied by the formation of viscous secretions, against which Sinupret is ineffective.

**Sinuforte Herbal** (Sinuforte^{®}, manufactured by EGIS Pharmaceuticals PLC, Hungary): lyophilisate of juice and extract of fresh tubers of European cyclamen; lyophilisate for a solution for intranasal administration (hemolytic index 1 : 6,000 to 1 : 12,000). It acts by strongly irritating the mucous membrane of the nasal cavity and eyes, which manifests as a pronounced burning sensation in the nose.

**Gelomyrtol Forte** (manufactured by G.POHL-BOSKAMP GmbH & Co.KG, Germany): enterosoluble capsules: Myrtol, standardized to a minimum content of limonene, cineole, alpha-pinene; a herbal preparation; the drug has an expectorant, mucolytic, antimicrobial, fungicidal, antioxidant, and deodorizing effect; it reduces bronchial discharge viscosity by changing pH; and facilitates sputum removal. *Pharmacokinetics:* absorbs well in the small intestine; reaches Cₘₐₓ in 2 hours; excreted by the lungs by facilitating ciliated epithelium activity; *indications:* acute and chronic bronchitis; sinusitis. The side effects of Gelomyrtol Forte include gastralgia (abdominal pain), dyspepsia, increased mobility of kidney stones and gallstones, as well as allergic reactions, which limits the drug's use.

**Rhinital** (manufactured by DEUTSCHE HOMOOPATHIE UNION-ARZNEIMITTEL GmbH & Co. KG, Germany): homeopathic tablets containing *Luffa operculata* (Cucurbitaceae family) in D4 dilution, *Galphimia glauca* (Malpighiaceae family) in D3 dilution and *Cardiospermum halicacabum* (Sapindaceae family) in D3 dilution, additionally containing lactose monohydrate, magnesium stearate, wheat starch. (Hereinafter, when indicating dilutions, the system proposed by S. Hahnemann is used [see, for example: Schwabe V. Homeopathic medicines (edited by V. Rybak). Moscow Scientific and Medical Society of Homeopathic Physicians, 1967], according to which the Latin letter D denotes a dilution of 10 times while the number after it denotes the number of repetitions of such dilutions, for example: D1 = 10× dilution, D3 = 1,000× dilution, etc., and the letter C = 100× dilution, for example: C1 = 100× dilution, C2 = 10,000× dilution, etc.)

Rhinital is prescribed in allergic rhinitis caused by plant pollen; year-round allergic rhinitis (as part of complex therapy).

**Aflubin** (manufactured by Artesan Alba, Austria): complex antiviral homeopathic drug produced as tincture and tablets; ingredients: yellow gentian (*Gentiana lutea*) D1, aconitum (*Aconitum napellus*) D6, redberry bryony (*Bryonia*) D6, ferric phosphate (*Ferrum phosphoricum*) D12, lactic acid (*Acidum sarcolacticum*) D12, ethyl alcohol (ethanol) 43%; indications: complex treatment of influenza, parainfluenza, and acute respiratory diseases to relieve the symptoms of these diseases, as well as for their prevention, both in routine and emergency settings; treatment of inflammatory and rheumatic diseases accompanied by joint pain syndrome.

**Euphorbium compositum** (manufactured by BIOLOGISCHE HEILMITTEL HEEL GmbH, Germany): homeopathic nasal spray; ingredients: Euphorbium D4, Pulsatilla pratensis (Pulsatilla) D2, Luffa operculata D2, Hydrargyrum biiodatum (Mercurius bijodatus) D8, Mucosa nasalis suis D8, Hepar sulfuris (Hepar sulfuris calcareum) DIO, Argentum nitricum DIO, Sinusitis-Nosode D13; excipients: benzalkonium chloride solution, sodium chloride, sodium dihydrogen phosphate dihydrate, sodium hydrogen phosphate dihydrate, purified water; indicated for rhinitis of various etiologies; chronic sinusitis, atrophic rhinitis, adenoids, eustachitis.

**Cinnabsin** (manufactured by DEUTSCHE HOMÖOPATHIE UNIONARZNEIMITTEL GmbH & Co. KG, Germany): tablets Cinnabaris D3, Hydrastis D3, Kalium bichromicum D3, Echinacea D1; excipients: lactose, magnesium stearate, wheat starch; indicated in the complex therapy of acute and chronic inflammation of the paranasal sinuses (sinusitis, frontal sinusitis, etc.); homeopathic drug with anti-inflammatory and anti-edematous effect that reduces increased secretion in the paranasal sinuses; facilitates nasal breathing; strengthens the immune system; the course of treatment for preventing relapses in chronic diseases is up to 2 months. **Ascinis** (manufactured by Richard Bittner, Ag, Austria): produced as drops for oral administration; ingredients: Armoracia (Russian horseradish), Cinnabaris (red mercury sulfide), Calcium sulfuricum (calcium sulfate), Kalium bichromicum (potassium dichromate); excipient: ethanol at a volume of 43% of the drug weight. This is a drug with complex homeopathic action mainly intended for treating nasal congestion of various etiologies, acute and chronic rhinosinusitis, rhinitis, pharyngitis, sinusitis; the treatment course in chronic diseases is 1 to 2 months.

Thus, the above nonrestrictive review of existing drugs for treating and preventing inflammatory diseases of the nasal cavity and paranasal sinuses shows that, despite the wide variety of existing drugs, the problem of searching for and developing new drugs based on natural components, including herbal components, for the treatment, including symptomatic, of both acute (ARS) and chronic rhinosinusitis (CRS) and nasopharyngitis still remains relevant due to the complexity of the treatment of acute inflammatory and exacerbated chronic, including allergic, rhinitis and rhinosinusitis.

Attempts to solve this problem using homeopathic medicine are known in the prior art. Such drugs have a number of advantages, including the absence of side effects (due to the use of very small doses of drugs that are devoid of toxic and allergic effects on the body), the natural origin of most components of homeopathic drugs, non-invasive administration, strictly individual selection of homeopathic drugs, and the ability to use such a homeopathic preparation as part of complex treatment. Due to the breadth of their pharmacological action and their low toxicity, natural components of homeopathic drugs have a mild complex effect and are less likely to cause side effects than synthetic agents, which allows for the long-term treatment of chronic diseases.

In particular, according to UA 96658 C2, 25.11.2011, A61K 9/16, A61K 36/185, A61P 37/08 (patent holder: National Pharmaceutical University, Ukraine), a homeopathic drug for treating and preventing allergic rhinitis is known marketed as granules, characterized by containing a matrix tincture of the juice of the above-ground part and tubers of *Cyclamen europaeum* in a certain dilution, as well as sugar powder.

According to UA 96658, the indications for the homeopathic drug are limited only to treating and preventing allergic rhinitis. This drug is not indicated for treating and preventing bacterial, viral, and mixed rhinitis, rhinosinusitis, and sinusitis.

According to RU 2152795 C1, 07.20.2000, A61K 35/78 (patent holders: Doctor N Limited Liability Company, Russia, and Natalia Petrovna Nechaeva, Russia), the prior art includes a homeopathic drug also known for the treatment of sinusitis, acute and chronic sinusitis with the viscous purulent to mucous fluid discharge produced as granules and containing *Hydrastis canadensis* and *Kalium bichromicum* characterized by that it further comprises *Pulsatilla pratensis, Thuja occidentalis, Phytolacca americana, Acidum silicium* taken in equal proportions with the following dilution of the components:
*Hydrastis canadensis-*C3*,*
*Kalium bichromicum-*C3*,*
*Pulsatilla pratensis-*C3,
*Thuja occidentalis-*C3,
*Phytolacca americana*-C3,
   and
*Acidum silicium-*C6.

According to RU 2152795, the drug is administered orally by placing the granules under the tongue until the granules are completely absorbed or by diluting the granules in 100 ml of liquid and administered orally; for successful treatment of maxillary sinusitis and sinusitis, the drug should be used for a long time: 8 granules 5 times a day for 3 months, repeating the course after a month.

In addition, according to RU 2152795, topical or intranasal administration cannot be used to enhance the anti-inflammatory effect and restore the mucous membrane of the nasal cavity and paranasal sinuses.

According to RU 2748548 C1, 05.26.2021, A61K 36/714, A61K 36/28, A61K 36/61, A61K 36/886, A61K 35/64, A61K 33/00, A61K 33/28, A61P 11/00 owned by the author of the present invention, a pharmaceutical (homeopathic) composition is known for treating and preventing upper respiratory tract diseases, in particular rhinosinusitis, proposed as the closest analogue containing alcoholic tinctures of *Aconitum napellus, Arnica montana, Pulsatilla pratensis,* alcoholic solution of *Apis mellifica* and homeopathic alcoholic solutions of *Kali bichromicum,* Mercurius solubilis (*Mercurius solubilis*)*, Antimonium tartaricum* in D3 to C30 dilutions with an equal volume ratio of the components. Furthermore, according to RU 2748548, the composition may also contain alcoholic tinctures of *Aloe vera* and/or Manuka honey with an equal volume ratio of components. Also known from RU 2748548 is a homeopathic drug for treating and preventing diseases of the upper respiratory tract containing the said pharmaceutical composition as an active component and a pharmaceutically acceptable topical carrier with volume-to-volume ratio of the active component to the pharmaceutically acceptable carrier of (0.5-15) : 100. Preferably, according to RU 2748548, the drug is produced as a nasal rinse solution, nasal drops, nasal spray, nasal gel, or nasal ointment.

According to RU 2748548, the drug is highly efficient as an anti-inflammatory, moisturizing, mucolytic agent, and a restorative agent, while its use for treating or preventing diseases of the upper respiratory tract provides strengthened local immunity of the upper respiratory tract mucous membrane by improving the function of the ciliated epithelium and stimulating the mucous membrane glands secretion, increasing the amount of protective enzymes, reducing congestion in the sinuses and nasopharynx, reducing bacterial activity and inflammation in the paranasal sinuses, improving blood flow in the tissues and stimulating metabolic processes, and activating granulation and epithelialization.

However, according to RU 2748548, the pharmaceutical composition and the drug have a number of drawbacks and limitations. Thus, according to RU 2748548, the pharmaceutical composition and the drug are intended only for treating and preventing upper respiratory tract diseases while, according to RU 2748548, treatment and prevention of lower respiratory tract diseases are not considered.

Furthermore, as is known from the prior art, these drugs suppress ciliated epithelium, slow down its action, and accelerate its death. This is shown in the paper by C Ruszak, JL Delavara, S Lozewicz, RJ Davies, Resp. Med. 1994, V 88, p. 89-101 (hereinafter referred to as *C. Ruszak et al., 1994*) through the example of the most common drugs used for treatment of upper respiratory tract diseases and the most common excipients used in the composition of such drugs. *C. Ruszak et al.,* 1994, examined the effect of drugs for topical administration such as xylometazoline and levocabastine usually used as nasal drops, as well as the effect of commonly used antimicrobial and antiviral treatment agents, mucolytics, and other groups of drugs on the functioning of the ciliated epithelium.

According to *C. Ruszak et al., 1994,* the ciliary beat frequency (CBF) is reduced by 69% by α-agonist xylometazoline hydrochloride (0.1% solution), by 13% by the H1 antagonist levocabastine, by 82% by commonly used antibiotic neomycin, by 33% to 47% in 5 hours by ribavirin, an antiviral agent, depending on the dose used, and by 18% to 100% by N-acetylcysteine, a commonly used mucolytic, depending on the dose used (see Table 1 on pp. 94-95 therein).

In addition, *C. Ruszak et al., 1994,* have given evidence that preservatives widely used in the composition of drugs such as chlorbutol, benzalkonium chloride with EDTA, propylene glycol, and thiomersal, also have a negative effect on the function of the upper respiratory tract epithelium cilia reducing the ciliary beat frequency by 100%, by 35%, by 30% to 100% (depending on the dose used) and by 100%, respectively (see ibid).

In this regard, it is important to create a means aimed at maintaining ciliated epithelium viability and enhancing its motor activity and, hence, increasing mucociliary clearance effectiveness.

Finally, a composition is required that has increased therapeutic and prophylactic efficacy in symptomatic and pathogenetic therapy of various inflammatory, allergic, post-traumatic (post-operative), atrophic, and hypertrophic processes in the upper respiratory tract mucous membrane, including the mucous membrane of the nasal cavity, paranasal sinuses, and pharynx, which more effectively improves mucociliary clearance in the upper respiratory tract mucous membrane. In addition, the task of expanding the range of topical drugs, in particular for intranasal use, for etiopathogenetic treatment or prevention of acute and chronic inflammatory diseases of the upper respiratory tract including diseases of bacterial, viral, allergic, and mixed etiology, remains relevant.

The present invention successfully solves these problems due to the pharmaceutical (homeopathic) composition of the invention for preventing or treating acute or chronic inflammatory diseases of the upper respiratory tract, including those with an allergic component, exacerbated chronic inflammatory diseases of the nasal cavity, nasopharynx, and paranasal sinuses, and pharynx, the composition comprising alcoholic tinctures of herbal components, including *Euphrasia officinalis, Allium cepa,* and *Arnica montana,* and, optionally, alcoholic tinctures of mineral components, including *Tartarus stibiatus* and *Hepar sulfur,* and an alcoholic solution of a component of animal origin, *Apis mellifica,* these being combined with a pharmaceutically acceptable carrier(- liquid base or solution)

The mentioned components are mixed with a saline and distilled water in a certain ratio. The pharmaceutical (homeopathic) composition of the invention can be used both for preventing and for symptomatic or pathogenetic therapy of various inflammatory, allergic, post-traumatic, in particular post-operative, atrophic, and hypertrophic processes of the upper respiratory tract.

It was unexpectedly discovered that, due to the mutual potentiation of its components, the pharmaceutical (homeopathic) composition of the invention ensures the achievement of a number of technical results, including:
- a super-cumulative (synergistic) improvement function of mucociliary clearance, a non-specific mechanism that provides local protection of the respiratory mucosa from external influences, in particular from exogenous pathogens (bacteria, viruses, etc.) and allergens,
- a super-cumulative inhibition of ciliated epithelial cytolysis due to inflammation of upper and lower respiratory tract,
- simplified therapy of acute and exacerbated chronic inflammatory diseases of the upper and lower respiratory tract, as well as allergic and post-traumatic conditions of nasal mucous membrane and paranasal sinuses,
- a super-cumulative restorative and anti-edematous effect, including in inflammatory, allergic, and post-traumatic conditions of the mucous membrane,
- a super-cumulative (synergistic) increase in the therapeutic activity of the pharmaceutical composition due to the combined use of natural herbal ingredients and, optionally, ingredients of animal and mineral origin in its formulation,
- simplified management, reduction, or inhibition symptoms of inflammatory and allergic of diseases of the upper and lower respiratory tract,
   and
- expanded range of topical pharmaceutical compositions intended for treating and preventing acute and chronic inflammatory and allergic diseases of the upper and lower respiratory tract with anti-inflammatory, anti-edematous, mucolytic, and restorative effects.

### Disclosure of the Invention

The present invention is aimed at providing a new agent that has increased effectiveness, in comparison with known analogues, in symptomatic and pathogenetic therapy of various inflammatory, allergic, post-traumatic (post-operative), atrophic, and hypertrophic processes of the mucous membrane of upper respiratory tract (nasal cavity, paranasal sinuses, pharynx), as well as the lower respiratory tract.

To explain the difference in the action of the treatment agent(s) of the invention currently available on the market, it is necessary to take into account the differences between the regular condition and abnormalities in the mucous membrane that occur in various inflammatory and allergic situations.

As is known, the mucous membrane of the nasal cavity and paranasal sinuses consists of three layers: smooth muscle and connective tissue fibers with inclusions of lymph nodes and epithelial surface layer consisting of epithelium (goblet cells that produce mucus and ciliated epithelium; the latter requires a moist mucous environment for its normal functioning (elimination)).

It is also well known that the main physiological function of the nasal mucosa is protective, i.e., it acts as the first line of defense of the upper and lower respiratory tract system, warming and purifying the inhaled air (10,000-20,000 liters of air per day). For this purpose, the mucous membrane should normally be moist, i.e., covered with mucus produced by goblet and cylindrical cells. The mucus also contains bacteriostatic substances such as lysozyme (muromidase), mucin, and lactoferrin, as well as secretory antibodies that, as was proven by Fleming, act as bacteriostatics that dissolve some microorganisms.

Disorders in mucus production as a secretory process can be represented as hyperproduction of mucus or a change in the qualitative composition of mucus and the presence of so-called pseudomucin (mucus becomes viscous, rubber-like).

Hypersecretion of mucus is a very common phenomenon in catarrhal changes of the mucous membrane and in vasomotor and secretory responses to reflex actions, both central and peripheral (when cold air affects the mucous membrane, in acute inflammation, flu, acute respiratory viral infections, etc.). Increased mucus production causes accumulation (water absorption), the cells gradually swell, the mucinogen turns into mucin, the upper part of the cell is destroyed, and the mucus is excreted into the nasal cavity lumen. During inflammation, goblet cells become hyperproductive and produce protective substances leading to an excessive production of mucus to accumulate in the nasopharynx.

Referring to the ciliated epithelium, it is known that each ciliated cell contains from 250 to 300 microtubules or cilia, each of which is in constant motion; they move in a strictly defined direction and make 6-8 beats per second. For the functioning of ciliated epithelium, the following conditions are required: a moist environment, T 28-32°C and nasal secretion pH of 5.5-6.5. With normal immunity and a fully functioning ciliated epithelium, all microbes (thousands of different pathogens every second) settle in the ciliated epithelium and are washed out by nasal secretions; therefore, in the overwhelming majority of cases, they do not harm us.

Changes in body temperature or mucus acidity (when using hypertonic or hypotonic solutions) lead to changed or discontinued cilia vibrations, which, in turn, leads to disruption of the entire mucociliary apparatus and chronic inflammatory changes in the mucous membrane or the development of dystrophic changes in it. Such changes are caused by constant hygienic operations, or simply regular mechanical washing of mucus from the mucous membrane; furthermore, the chemical composition of mucus is disrupted and changes in the nasal and paranasal microflora homeostasis occur. This results in triggering the chronic inflammation mechanisms.

Many of disadvantages of the prior art are eliminated by the pharmaceutical composition of the invention for preventing or treating acute or chronic diseases of the upper respiratory tract, exacerbated chronic inflammatory diseases of the nasal cavity, nasopharynx, and paranasal sinuses, and pharynx, characterized in that it comprises alcoholic tinctures of herbal components, including *Euphrasia officinalis, Allium cepa,* and *Arnica montana,* combined with a physiologically acceptable carrier.

*Euphrasia officinalis* is a drug obtained from the drug eyebright used in homeopathic medicine both as granules for internal use and solutions, including for topical use. Indications for the use of *Euphrasia officinalis* include inflammatory eye diseases, such as conjunctivitis, iritis (inflammation of the iris), hay fever, traumatic eye injury, as well as chronic eye diseases, such as cataracts, dry eye syndrome, and increased intraocular pressure. *Euphrasia officinalis* is also indicated in catarrhal and seasonal viral infections, pollinosis, hay fever, bronchitis developing as a complication of acute respiratory viral infections, inflammatory processes in the gastrointestinal tract, sycosis, symptoms of nasal cancer, as well as menstrual irregularities in women and prostatitis in men.

According to the present invention, *Euphrasia officinalis* is preferably, but not exclusively, used in the form of an alcoholic tincture *or an alcoholic solution* in dilution in a D3 to C30 dilution.

*Allium cepa* is a drug obtained from an onion (bulb) used in homeopathic medicine for treating viral, bacterial, and fungal infections, and for conjunctivitis and rhinitis, including allergic rhinitis. It is also used for treating pharyngitis manifested as a burning sensation in the throat and accompanied by a hard cough and/or sore throat which radiates to the ears, gastritis with stabbing pain which progresses in the morning, stabbing pain in the rectum, inflammatory processes in the intestines, cystitis, urolithiasis, and, finally, for treating phantom pain in amputated extremities.

According to the present invention, *Allium cepa* is preferably, but not exclusively, used as an alcoholic tincture *or an alcoholic solution* in dilution in a D3 to C30 dilution.

*Arnica montana* is a drug obtained from mountain arnica. In healthcare, an alcoholic tincture of inflorescences is used as a hemostatic agent in uterine and nasal bleeding, insufficient uterus involution after childbirth and inflammatory processes of the genital area in obstetric and gynecological practice, as well as in edema and cardiac weakness. Arnica has been shown to have beneficial effects in treating angina pectoris and heart failure. Animal experiments have shown that Arnica treatment agents cause increased contractions of the uterine muscles and have a stimulating effect on the heart and central nervous system. Furthermore, they dilate the coronary vessels of the isolated heart, have a choleretic effect, and lower the blood cholesterol due to cynarin present in Arnica.

Arnica treatment agents are intended for topical use in bruises and hematomas, carbuncles, furuncles, and abscesses as a resolvent and counter-irritant agent.

*Arnica montana* is known as a cardiant and hemostatic agent in various injuries used as a tincture of dried rhizomes with roots or essence of fresh flowering grass. In conventional medicine, Arnica is used in fever, as a diuretic, diaphoretic, astringent agent in gastrointestinal disorders, as an anti-inflammatory agent in gynecological diseases, in bronchitis and flu, as well as in epilepsy and concussion. For topical use, an aqueous infusion of mountain arnica anthodium was applied in skin rashes (especially on the lips), furunculosis, ulcers, bruises, rheumatism, gout, neuralgia, lumbago, and toothache. Tincture of mountain arnica roots for internal use was administered in cardiac angiospasms, cardiosclerosis, myocarditis. It is also used as a stimulating agent, in bruises, contusions, minor wounds, and abscesses.

In homeopathic medicine, Mountain Arnica is used as part of complex therapy for treating injuries (bruises, wounds, fractures, sports injuries, etc.), hemorrhages, consequences of childbirth, cephalohematoma of newborns; in pre- and postoperative preparation of patients; as part of complex therapy for treating post-traumatic pain syndrome, stress-related myalgia (muscle pain), prolonged muscle tension, etc.; for hoarseness due to vocal cord strain in lecturers, singers, speakers, and other people whose voice is important in their profession; in dentistry; for treating inflammatory diseases of the oral cavity, such as stomatitis and gingivitis; in ophthalmology, for treating subconjunctival hemorrhage, retinopathy, and age-related macular degeneration; in gynecology, as part of complex therapy in episiotomy, cesarean section, and preparation for childbirth; in dermatology, as part of complex therapy for treating couperose and rosacea upon symmetrical location of inflamed areas.

Arnica is also used in otolaryngology for preventing or treating epistaxis and for treating and mitigating the consequences of tissue and mucous membrane trauma after procedures and surgeries such as tonsillectomy.

According to the present invention, *Arnica montana* is preferably, but not exclusively, used as an alcoholic tincture or an alcoholic solution in dilution in a D3 to C30 dilution.

Preferably, but not limited to these embodiments of the invention, the physiologically acceptable carrier is a saline (0.9% sodium chloride solution) or water, such as (but not limited to) distilled water. In alternative embodiments of the invention, but not exclusively, the physiologically acceptable carrier may be a thickener and/or a gelling base as disclosed herein below. In other, also nonexclusive, embodiments of the invention, the physiologically acceptable carrier may be an ointment base as disclosed below in this description.

It is also preferable, but not limited to these embodiments of the invention, that the volume ratio of the *Euphrasia officinalis, Allium cepa,* and *Arnica montana* alcoholic tinctures on the one part and the physiologically acceptable carrier on the other part is from 0.5 ml : 100 ml to 15 ml : 100 ml.

It is also preferable, but not limited to these embodiments of the invention, that the pharmaceutical composition of the invention further comprises one or more components selected from an alcoholic tincture or alcoholic solution of *Tartarus stibiatus,* an alcoholic tincture or alcoholic solution of *Hepar sulfur,* and an alcoholic solution of *Apis mellifica.*

*Tartarus stibiatus* (tartar emetic) is a tartrate of antimony and potassium (C₄H₄O₆(SbO)K_{1/2}*H₂O). *Tartarus stibiatus* provides a beneficial effect on the mucous membranes of the respiratory tract and gastrointestinal tract; therefore, in homeopathic medicine, it is used in bronchial asthma, gout, skin diseases, and gastrointestinal tract disorders.

According to the present invention, *Tartarus stibiatus* is preferably, but not exclusively, used as *solution* in a D3 to C30 dilution.

*Hepar sulfur* (hepar sulphuris calcareum) is a mixture of calcium polysulfides and calcium phosphate obtained by calcining for a long time equal weight parts of fine powder from the inside of oyster shells and sulfur flower in a crucible. *Hepar sulfur* is stored in a tightly closed glass vial protected against water. In homeopathic medicine, *Hepar sulfur* is used in inflammatory processes and/or suppurations of any localization, in particular, in dermatology, otolaryngology, pulmonology, ophthalmology, pediatrics, etc.

According to the present invention, *Hepar sulfur* is preferably, but not exclusively, used as solution in dilution in a D3 to C30 dilution.

*Apis mellifica* is a homeopathic agent obtained from the extract of honey bees and has a very complex composition including, but not limited to, enzymes (phospholipase A2 and hyaluronidase), peptides (melitin, apamin, MCD peptide), free amino acids, etc. In homeopathic medicine, *Apis mellifica* is used to treat inflammatory diseases of various localizations accompanied by severe swelling and/or pain syndrome. In particular, in ophthalmology, *Apis mellifica* is used for treating conjunctivitis; in rheumatology, for treating post-traumatic hydrarthrosis and acute arthritis; in urology, for treating interstitial cystitis with decreased urine output, increased protein content in the urine, swelling without thirst; in gynecology, for treating ovarian inflammation and for relieving pain associated with milk production and the development of ovarian cysts; in neurology, for treating headaches or vasomotor migraine; in otolaryngology, for treating edematous rhinosinusitis, pharyngitis, and tonsillitis of non-streptococcal origin with swelling of the uvula, as well as for relieving a sore throat caused by drinking cold water.

According to the present invention, *Apis mellifica* is preferably, but not limited to these dilutions, used as an alcoholic solution in a D3 to C30 dilution.

Preferably, but not limited to these embodiments of the invention, the composition of the present invention contains, in addition to alcoholic tinctures or *alcoholic solutions of Euphrasia officinalis, Allium cepa,* and *Arnica montana,* at least one component selected from alcoholic tinctures *alcoholic solutions of Tartarus stibiatus, Hepar sulfur,* and an alcoholic solution of *Apis mellifica* in an equal volume ratio of the components. In a more preferred, but also non-limiting embodiment of the invention, the composition of the invention comprises, in addition to the alcoholic tinctures of *Euphrasia officinalis, Allium cepa,* and *Arnica montana,* at least two components selected from the alcoholic tinctures of *Tartarus stibiatus, Elepar sulfur,* and an alcoholic solution of *Apis mellifica* in an equal volume ratio of the components. In the most preferred, but also non-limiting embodiment of the invention, the composition of the invention comprises alcoholic tinctures or alcoholic solutions of *Euphrasia officinalis, Allium cepa, Arnica montana, Tartarus stibiatus,* and *Hepar sulfur,* as well as an alcoholic solution of *Apis mellifica* in an equal volume ratio of the components.

Preferably, the pharmaceutical (homeopathic) composition of the invention is produced as a nasal rinse solution, nasal drops, or nasal spray. In alternative embodiments of the invention, the pharmaceutical (homeopathic) composition of the invention may be produced as a nasal gel, nasal ointment, granules, tablets, capsules, dragee, pills, or other dosage forms well known to a person skilled in the art. In all the above cases, the pharmaceutical (homeopathic) composition of the invention contains the above homeopathic dilutions of alcoholic tinctures of *Euphrasia officinalis, Allium cepa,* and *Arnica montana,* as well as, optionally, the abovementioned homeopathic dilutions of alcoholic tinctures of *Tartarus stibiatus* and *Hepar sulfur* and the abovementioned homeopathic dilutions of alcoholic solution of *Apis mellifica,* together with an excipient or excipients traditionally used in this field to produce the corresponding dosage form. Specific (non-limiting) examples of such excipients include one or more stabilizers, pH regulators, preservatives, thickeners, gelling bases (for a nasal gel composition), or ointment (ointment-forming) bases (for a nasal ointment composition).

The pH adjusters that can be used in the present invention include, but are not limited to, substances known to a person skilled in the art such as sodium bicarbonate, sodium hydroxide, potassium hydroxide, boric acid, disodium hydrogen phosphate, sodium dihydrogen phosphate, and polar organic amines, including, but not limited to, diethylamine, diisopropylamine, triethylamine, and triethanolamine. It is clear to a person skilled in the art that mixtures of these components or other components known to a person skilled in the art and used in this field can also be used as pH regulators in the compositions of the invention.

Preservatives that may be used in accordance with the present invention include, but are not limited to, substances known to a person skilled in the art such as parahydroxybenzoic acid derivatives (parabens) including, but not limited to, methyl parahydroxybenzoate and propyl parahydroxybenzoate. It is clear to a person skilled in the art that other substances used in this field as preservatives may also be used.

According to the present invention, any pharmaceutically acceptable excipients known in this field and used for this purpose can be used as thickeners/gelling bases; for example, but not limited to, synthetic or semi-synthetic polymer compounds, polyacrylate resins and resins based on polyacrylate copolymers; for example (without limitation), polyacrylic acid and copolymers of acrylic and methacrylic acids marketed under the trade name CARBOPOL, in particular CARBOPOL 934, 940 and 941, and EUDRAGIT E, L, S, RL and RS; cellulose and cellulose derivatives; for example (without limitation), alkyl celluloses, in particular methyl, ethyl, and propyl celluloses; hydroxyalkyl celluloses, in particular hydroxypropyl cellulose, hydroxypropyl alkyl cellulose such as hydroxypropyl methyl cellulose, acylated celluloses, in particular cellulose acetates, cellulose acetate phthalate and their salts such as sodium carboxymethyl cellulose; polyvinyl resins, including polyvinyl acetates and alcohols, as well as other polymer compounds, including alginates; for example, alginic acid and its salts, in particular sodium alginate and propylene glycol alginate. In addition, combinations of these components or other components known to a person skilled in the art and used in this field can be used as gelling bases in the compositions of the invention.

In accordance with the present invention, any pharmaceutically acceptable excipients used in this field in the appropriate capacity can be used as ointment (ointment-forming) bases. In one of the most preferred embodiments of the invention, but not limited thereto, the ointment base contains cetyl alcohol, cholesterol, white petrolatum, methyl parahydroxybenzoate, propyl parahydroxybenzoate, and water for injection. In other, also non-limiting, embodiments of the invention, the ointment base may comprise another fat-soluble or water-soluble base well known to a person skilled in the art. Non-limiting examples of a fat-soluble base include petrolatum. This base does not cause severe irritation and has no odor, which provides an excellent protective effect for the nasal mucosa. Non-limiting examples of a water-soluble base include a base containing a macrogol. Such bases are able to perfectly absorb and remove water discharge. In addition, combinations of these components or other components known to a person skilled in the art and used in this field can be used as ointment (ointment-forming) bases in the compositions of the invention.

Other excipients that can also be used in one or another particular embodiment of the composition of the invention are well known to persons skilled in the art and include, but are not limited to, preservatives, stabilizers, pH regulators, plasticizers, etc. Non-limiting examples of such excipients are given in the Handbook of Pharmaceutical Excipients, 3rd ed. (Kibbe, ed. (2000), Washington: American Pharmaceutical Association) or in E. I. Sakanyan, N. S. Teryoshina, M. N. Lyakina and O. A. Naumova. Excipients for Homeopathic Medicines: Characteristics and Quality Requirements-Pharmaceutical Chemistry Journal, volume 54, pages 208-212 (2020*),* which are incorporated herein by reference.

The said problems within the present invention are also solved while the technical results are achieved by using the said composition for symptomatic and pathogenetic treatment and prevention of acute and exacerbated chronic inflammatory, allergic, atrophic, and hypertrophic diseases of the upper respiratory tract, the said use including the administration of the said pharmaceutical composition to the appropriate patient in an effective amount.

Moreover, within the present invention, unexpected data were obtained indicating that the composition of the invention improves mucociliary clearance of the ciliated epithelium of not only the upper but also of the lower respiratory tract (larynx, trachea, bronchi, and lungs).

To determine the efficiency of mucociliary clearance, the authors of the present invention measured the frequency (Frequency) and period (Time) of the upper respiratory tract ciliated epithelium cilia beating using methods for determining these parameters that are well known to persons skilled in the art and have been repeatedly described in the prior art. The authors of the invention took into account that sufficient functioning of the ciliated epithelium cilia is the most important parameter of the effectiveness of mucociliary clearance (Duchateau GSMJE, Graamans K., Zuidema J., Merkus FWHM.-Laryngoscope 1985; 95: 854-859), and, therefore, they measured the percentage of ciliated epithelium cells with motile cilia (Movement). Non-limiting (illustrative) examples of methods for determining the parameters of the upper respiratory tract ciliated epithelium cilia beating are given in V. V. Shabalin et al., Biophysical Methods for Studying Mucociliary Transport of the Normal and Pathological Upper and Lower Respiratory Tract.-See in Problems of Therapeutic and Surgical Pulmonology, collection of conference materials, Saint Petersburg, 1997, pp. 44-45*.*

It is well known to a person skilled in the art that there are other reliable methods for studying mucociliary clearance that are not mentioned in this paper by V. V. Shabalin, including other reliable methods for determining the percentage of ciliated epithelial cells with motile cilia and the ciliated epithelium cilia beating frequency that can also be used to study and evaluate the effect of the composition of the invention on mucociliary clearance.

As non-limiting (illustrative) examples of such methods, the methods disclosed in patents RU 2254805 C1, 27.06.2005, A61B 5/11, G01N 33/483 and RU 2410028 C2, 27.01.2011, A61B 5/11, G01N 33/483, which are incorporated herein by reference, can be mentioned. Other examples of such study methods are well known to persons skilled in the art and include, but are not limited to, visual assessment methods (such as methods according to Bisgaard H., Pedersen M., Clinical Allergy 1987; 17: 95-103*;* Stafanger G., Bisgaard H. et al., Eur. J. Respir. Dis. 1987; 70: 157-162), audio methods (such as methods according to Bleeker J.D., Hoeksema PE., Acta Ololaryngol. 1971; 71: 426-429), stroboscopic methods (e.g., methods according to Gray J., Proc. R. Soc. 1930; 107: 313), methods using video or photographic recording (such as methods according to Rylander R., Am. Rev. Respir. Dis. 1966; 93 (suppl.): 67-85*,* Dalhamn T., Acta Physiol Scand 1955; 33: 1-5*,* Sanderson M.J., Sleigh M.A., J. Cell Sci 1981; 47: 331-347*,* and Rautiainen M., Matsune S., et al., Acta Otolaryngol. 1992; 112: 845-851), photovoltaic methods (e.g., methods according to Rutland J, Griffin W, Cole PJ, Br J Clin Pharmacol 1982; 13: 679-683*,* Yager J, Chen TM, Dulfano MJ., Chest 1978; 73: 627-633*,* Yager JA, Ellman H, Dulfano MJ., Am Rev Respir Dis 1980; 121: 661-665*,* Tamaoki J., Kondo M., Takizawa T., Am Rev Respir Dis 1989; 139: 441-445*,* Dalhamn T., Rylander R., Nature 1962; 196: 592-593*,* Kennedy J.R., Duckett K.E., Exp. Cell Res 1981; 135: 147-156*,* Sanderson M.J., Dirksen E.R., Cell Motil. 1985; 5: 267-292*,* etc.), methods using laser scattering spectroscopy (e.g., methods according to Lee W.I., Verdugo P, Ann. Biomed. Eng. 1977; 5: 248-259) and analogue contrast enhancement techniques (such as methods according to Devalia J.L., Sapsford R.J., et al., Respir. Med. 1990; 84: 303-312*;* Devalia J.L., Sapsford R.J., et al., Pulm. Pharmacol. 1992; 5: 257-263*;* Devalia J.L., Sapsford R.J., et al., Eur. Respir. J. 1993; 6: 1308-1316*;* Devalia J.L., Sapsford R.J., et al., Am J Respir Cell Mol Biol 1992; 7: 270-277).

### Example 1. Comparative Study of the Drug's Effectiveness.

The effect of the composition of the invention on the performance of the ciliated epithelium cilia and the lifespan of the ciliated epithelium was studied in comparison with separate components (*Apis mellifica, Eupharsia officinalis, Allium cepa, Tatrarus stibiatus, Hepar sulphur,* and *Arnica montana* separately), with the composition according to patent RU 2748548 and with an isotonic NaCl solution (0.9% solution).

The study included patients with acute and chronic rhinosinusitis, as well as patients without a history of otolaryngological disorders.

It is well known to persons skilled in the art that the optimal medium for the ciliated epithelium functioning is a saline (0.9% NaCl solution) and that this medium allows the ciliated epithelium cells to move synchronously and live the longest; therefore, ciliated epithelium treated with a saline was used as a control.

Tables 1-6 below show the results of the study for individual components of the composition of the invention (for *Apis mellifica,* for *Eupharsia officinalis,* for *Allium cepa,* for *Tartarus stibiatus,* for *Hepar sulphur,* and for *Arnica montana,* respectively) taken in various dilutions (ranging from D3 to C30).

The results include the following parameters:
Frequency 1, Hz-the beating frequency of the ciliated epithelium cilia in the test sample,
Frequency 2, Hz-the beating frequency of the ciliated epithelium cilia in the control (isotonic NaCl solution),
Time 1, min-lifespan of the ciliated epithelium cilia in the test sample,
Time 2, min-lifespan of the ciliated epithelium cilia in the control.

Table 7 shows the results of the study for the comparator drug (alcoholic tincture of belladonna (*Atropa belladonna*)) taken in various dilutions ranging from D3 to C30.

Tables 8-12 show the results of the study for the closest analogue (the treatment agent according to RU 2748548) using various dilutions of its components.

Tables 13-22 show the results of the study for the composition of the invention obtained using various dilutions of its components.

**Table 1. Results for Apis mellifica (alcoholic solution), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient B. | 9 | tincture | 6.553 | 6.787 | 18 | 28 |
| Patient C. | 11 | D3 | 6.163 | 8.028 | 27 | 29 |
| Patient K. | 12 | C3 | 7.812 | 8.663 | 25 | 27 |
| Patient L. | 8 | C12 | 5.075 | 7.1 | 3 | 5 |
| Patient N. | 8 | C30 | 4.801 | 5.466 | 17 | 34 |
| Average values | | | 6.0808 | 7.2088 | 18 | 24.6 |

**Table 2. Results for Eupharsia officinalis (alcoholic tincture), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient R. | 14 | tincture | 5.521 | 6.787 | 25 | 30 |
| Patient C. | 9 | D3 | 5.756 | 7.49 | 29 | 31 |
| Patient B. | 17 | D6 | 6.393 | 7.62 | 19 | 25 |
| Patient S. | 17 | C6 | 5.013 | 5.27 | 27 | 30 |
| Patient T. | 17 | C30 | 4.875 | 8.244 | 18 | 20 |
| Average values | | | 5.5116 | 7.0822 | 23.6 | 27.2 |

**Table 3. Results for Allium cepa (alcoholic tincture), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient K. | 17 | tincture | 5.575 | 7.507 | 5 | 8 |
| Patient G. | 17 | D6 | 5.579 | 6.339 | 15 | 36 |
| Patient S. | 9 | C3 | 5.528 | 6.109 | 18 | 32 |
| Patient B. | 14 | C12 | 5.945 | 7.367 | 22 | 27 |
| Patient A. | 11 | C30 | 5.528 | 5.677 | 3 | 4 |
| Average values | | | 5.631 | 6.5998 | 12.6 | 21.4 |

**Table 4. Results for Tartarus stibiatus (alcoholic tincture), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient D. | 17 | D3 | 5.177 | 5.543 | 27 | 62 |
| Patient V. | 14 | D6 | 5.012 | 7.733 | 20 | 41 |
| Patient A. | 6 | C3 | 6.503 | 7.396 | 28 | 33 |
| Patient K. | 14 | C6 | 5.283 | 5.876 | 20 | 52 |
| Patient G. | 16 | C30 | 4.092 | 5.973 | 28 | 30 |
| Average values | | | 5.2134 | 6.5042 | 24.6 | 43.6 |

**Table 5. Results for Hepar sulphur (alcoholic tincture), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient F. | 11 | D3 | 6.064 | 7.188 | 25 | 27 |
| Patient M. | 17 | D6 | 5.224 | 5.335 | 18 | 45 |
| Patient Z. | 17 | C3 | 5.665 | 5.759 | 14 | 34 |
| Patient B. | 16 | C12 | 4.227 | 5.995 | 12 | 18 |
| Patient I. | 17 | C30 | 4.777 | 5.063 | 10 | 12 |
| Average values | | | 5.1914 | 5.868 | 15.8 | 27.2 |

**Table 6. Results for Arnica montana (alcoholic tincture), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient A. | 10 | tincture | 5.279 | 7.534 | 18 | 27 |
| Patient I. | 15 | D6 | 8.507 | 10.465 | 23 | 28 |
| Patient P. | 17 | C6 | 5.877 | 6.308 | 24 | 34 |
| Patient S. | 17 | C12 | 7.075 | 8.167 | 19 | 42 |
| Patient V. | 15 | C30 | 6.107 | 6.758 | 23 | 26 |
| Average values | | | 6.569 | 7.8464 | 21.4 | 31.4 |

**Table 7. Results for Atropa belladonna (alcoholic tincture), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient B. | 10 | D6 | 5.773 | 6.334 | 7 | 9 |
| Patient R. | 15 | C3 | 4.479 | 4.638 | 19 | 21 |
| Patient M. | 12 | C6 | 5.829 | 7.525 | 27 | 53 |
| Patient K. | 11 | C12 | 5.839 | 6.094 | 22 | 29 |
| Patient B. | 17 | C30 | 5.076 | 6.066 | 9 | 12 |
| Average values | | | 5.3992 | 6.1314 | 16.8 | 24.8 |

**Table 8. Results for Composition 1 according to patent RU 2748548 (Aconitum napellus + Arnica montana + Pulsatilla pratensis + Apis mellifica + Kali bichromicum + Mercurius solubilis + Antimonium tartaricum), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient F. | 14 | D3 | 7.045 | 8.097 | 27 | 32 |
| Patient E. | 12 | D6 | 0 | 0 | 1 | 2 |
| Patient J. | 9 | C3 | 5.98 | 6.394 | 14 | 16 |
| Patient N. | 12 | C12 | 5.447 | 7.852 | 29 | 44 |
| Patient M. | 13 | C30 | 6.71 | 7.037 | 30 | 44 |
| Average values | | | 5.0364 | 5.876 | 20.2 | 27.6 |

**Table 9. Results for Composition 2 according to patent RU 2748548 (Aconitum napellus + Arnica montana + Pulsatilla pratensis + Apis mellifica + Kali bichromicum + Mercurius solubilis + Antimonium tartaricum), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient S. | 12 | D3 | 7.633 | 9.323 | 16 | 19 |
| Patient R. | 15 | D6 | 0 | 0 | 3 | 5 |
| Patient V. | 15 | C6 | 6.729 | 8.214 | 29 | 42 |
| Patient F. | 14 | C12 | 5.938 | 6.522 | 16 | 19 |
| Patient L. | 16 | | 7.628 | 9.811 | 15 | 18 |
| Average values | | C30 | 5.5856 | 6.774 | 15.8 | 20.6 |

**Table 10. Results for Composition 3 according to patent RU 2748548 (Aconitum napellus + Arnica montana + Pulsatilla pratensis + Apis mellifica + Kali bichromicum + Mercurius solubilis + Antimonium tartaricum + Aloe vera), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient S. | 8 | D6 | 6.814 | 9.417 | 13 | 15 |
| Patient P. | 8 | C3 | 6.864 | 7.01 | 5 | 18 |
| Patient A. | 9 | C6 | 6.324 | 7.418 | 11 | 18 |
| Patient C. | 11 | C12 | 0 | 0 | 29 | 33 |
| Patient N. | 17 | C30 | 5.249 | 8.924 | 21 | 26 |
| Average values | | | 5.0502 | 6.5538 | 15.8 | 22 |

**Table 11. Results for Composition 4 according to patent RU 2748548 (Aconitum napellus + Arnica montana + Pulsatilla pratensis + Apis mellifica + Kali bichromicum + Mercurius solubilis + Antimonium tartaricum + Manuka honey), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient M. | 17 | D6 | 0 | 0 | 6 | 9 |
| Patient N. | 17 | C3 | 6.315 | 7.464 | 29 | 38 |
| Patient L. | 9 | C6 | 7.205 | 9.72 | 16 | 17 |
| Patient T. | 4 | C12 | 6.274 | 9.674 | 26 | 27 |
| Patient O. | 12 | C30 | 0 | 0 | 4 | 7 |
| Average values | | | 3.9588 | 5.3716 | 16.2 | 19.6 |

**Table 12. Results for Composition 5 according to patent RU 2748548 (Aconitum napellus + Arnica montana + Pulsatilla pratensis + Apis mellifica + Kali bichromicum + Mercurius solubilis + Antimonium tartaricum + Manuka honey + Aloe vera), D3-C30 dilution**

| **Name** | **Age** | **Dilution** | **Frequency 1, Hz** | **Frequency 2, Hz** | **Time 1, min** | **Time 2, min** |
|---|---|---|---|---|---|---|
| Patient L. | 14 | D3 | 6.808 | 7.321 | 18 | 20 |
| Patient K. | 16 | D6 | 0 | 0 | 7 | 9 |
| Patient M. | 8 | C6 | 6.601 | 7.834 | 19 | 20 |
| Patient M. | 15 | C12 | 7.894 | 8.043 | 31 | 56 |
| Patient A. | 14 | C30 | 8.693 | 10.082 | 24 | 26 |
| Average values | | | 5.9992 | 6.656 | 19.8 | 26.2 |

**Table 13. Results for the test composition, Composition 1 (Tartarus stibiatus (alcoholic tincture), D3 dilution + Hepar sulphur (alcoholic tincture), D3 dilution + Apis mellifica (alcoholic solution), D6 dilution + Eupharsia officinalis (alcoholic tincture), D3 dilution + Allium cepa (alcoholic tincture), D3 dilution + Arnica montana (alcoholic tincture), D6 dilution)**

| **Name** | **Age** | **Abnormal or normal** | **Frequency 1, Hz** | **Frequency 2, Hz** | **Time 1, min** | **Time 2, min** |
|---|---|---|---|---|---|---|
| Patient R. | 13 | Normal | 6.685 | 9.756 | 45 | 29 |
| Patient B. | 17 | Chronic rhinitis | 9.049 | 8.894 | 26 | 23 |
| Patient C. | 14 | Normal | 8.449 | 9.36 | 35 | 32 |
| Patient S. | 17 | Chronic rhinitis | 8.166 | 7.341 | 22 | 12 |
| Patient T. | 14 | Chronic rhinitis | 9.528 | 10.14 | 20 | 12 |
| Patient K. | 11 | Acute rhinitis | 0 | 0 | 21 | 15 |
| Patient G. | 13 | Normal | 9.079 | 7.631 | 21 | 12 |
| Patient S. | 17 | Chronic rhinitis | 8.679 | 9.535 | 26 | 12 |
| Patient B. | 15 | Chronic rhinitis | 6.272 | 4.991 | 45 | 33 |
| Patient J. | 17 | Chronic rhinitis | 7.14 | 6.317 | 46 | 33 |
| Average values | | | 7.3047 | 7.3965 | 30.7 | 21.3 |

**Table 14. Results for the test composition, Composition 2 (Tartarus stibiatus (alcoholic tincture), C3 dilution + Hepar sulphur (alcoholic tincture), C3 dilution + Apis mellifica (alcoholic solution), C12 dilution + Eupharsia officinalis (alcoholic tincture) + Allium cepa (alcoholic tincture), C6 dilution + Arnica montana (alcoholic tincture))**

| **Name** | **Age** | **Abnormal or normal** | **Frequency 1, Hz** | **Frequency 2, Hz** | **Time 1, min** | **Time 2, min** |
|---|---|---|---|---|---|---|
| Patient A. | 11 | Acute rhinitis | 0 | 0 | 9 | 4 |
| Patient N. | 15 | Chronic rhinitis | 7.04 | 6.537 | 34 | 26 |
| Patient K. | 15 | Normal | 5.499 | 6.707 | 49 | 35 |
| Patient U. | 6 | Normal | 7.462 | 5.413 | 40 | 30 |
| Patient S. | 15 | Chronic rhinitis | 5.98 | 6.002 | 36 | 39 |
| Patient P. | 13 | Acute rhinitis | 2.265 | 1.249 | 23 | 12 |
| Patient D. | 17 | Chronic rhinitis | 6.177 | 5.543 | 69 | 62 |
| Patient V. | 14 | Normal | 6.012 | 7.733 | 45 | 41 |
| Patient A. | 6 | Chronic rhinitis | 8.503 | 7.396 | 41 | 33 |
| Patient K. | 14 | Chronic rhinitis | 6.283 | 5.876 | 59 | 52 |
| Average values | | | 5.5221 | 5.2456 | 40.5 | 33.4 |

**Table 15. Results for the test composition, Composition 3 (Tartarus stibiatus (alcoholic tincture), D3 dilution + Hepar sulphur (alcoholic tincture), C30 dilution + Apis mellifica (alcoholic tincture), C30 dilution + Eupharsia officinalis (alcoholic tincture), D6 dilution + Allium cepa (alcoholic tincture) + Arnica montana (alcoholic tincture), C30 dilution)**

| **Name** | **Age** | **Abnormal or normal** | **Frequency 1, Hz** | **Frequency 2, Hz** | **Time 1, min** | **Time 2, min** |
|---|---|---|---|---|---|---|
| Patient G. | 16 | Normal | 6.092 | 5.973 | 40 | 30 |
| Patient F. | 11 | Chronic rhinitis | 7.064 | 7.188 | 39 | 27 |
| Patient M. | 17 | Chronic rhinitis | 7.284 | 5.335 | 54 | 45 |
| Patient Z. | 17 | Normal | 5.607 | 5.759 | 56 | 34 |
| Patient B. | 16 | Chronic rhinitis | 6.245 | 5.995 | 13 | 18 |
| Patient P. | 17 | Chronic rhinitis | 7.775 | 5.063 | 20 | 12 |
| Patient B. | 10 | Chronic rhinitis | 7.773 | 6.334 | 24 | 9 |
| Patient R. | 15 | Chronic rhinitis | 5.979 | 4.638 | 56 | 21 |
| Patient M. | 12 | Normal | 6.829 | 7.525 | 125 | 53 |
| Patient K. | 11 | Acute rhinitis | 6.839 | 6.094 | 38 | 29 |
| Average values | | | 6.7487 | 5.9904 | 46.5 | 27.8 |

**Table 16. Results for the test composition, Composition 4 (Tartarus stibiatus (alcoholic tincture), C12 dilution + Hepar sulphur (alcoholic tincture), C6 dilution + Apis mellifica (alcoholic solution), C3 dilution + Eupharsia officinalis (alcoholic tincture), C30 dilution + Allium cepa (alcoholic tincture), C12 dilution + Arnica montana (alcoholic tincture), C3 dilution)**

| **Name** | **Age** | **Abnormal or normal** | **Frequency 1, Hz** | **Frequency 2, Hz** | **Time 1, min** | **Time 2, min** |
|---|---|---|---|---|---|---|
| Patient B. | 17 | Chronic rhinitis | 0 | 0 | 33 | 12 |
| Patient I. | 15 | Chronic rhinitis | 6.832 | 0 | 23 | 14 |
| Patient N. | 15 | Acute rhinitis | 6.634 | 5.272 | 57 | 46 |
| Patient S. | 13 | Chronic rhinitis | 6.512 | 5.297 | 27 | 10 |
| Patient I. | 14 | Normal | 8.617 | 9.327 | 36 | 26 |
| Patient K. | 17 | Normal | 9.548 | 7.315 | 65 | 28 |
| Patient D. | 11 | Normal | 5.456 | 6.717 | 25 | 33 |
| Patient P. | 9 | Normal | 6.24 | 6.863 | 41 | 29 |
| Patient B. | 15 | Chronic rhinitis | 0 | 0 | 17 | 14 |
| Patient C. | 11 | Normal | 6.861 | 7.552 | 31 | 20 |
| Average values | | | 5.67 | 4.8343 | 35.5 | 23.2 |

**Table 17. Results for the test composition, Composition 5 (Tartarus stibiatus (alcoholic tincture), C3 dilution + Hepar sulphur (alcoholic tincture), C12 dilution + Apis mellifica (alcoholic solution), D3 dilution + Eupharsia officinalis (alcoholic tincture), C3 dilution + Allium cepa (alcoholic tincture), C12 dilution + Arnica montana (alcoholic tincture), C30 dilution)**

| **Name** | **Age** | **Abnormal or normal** | **Frequency 1, Hz** | **Frequency 2, Hz** | **Time 1, min** | **Time 2, min** |
|---|---|---|---|---|---|---|
| Patient S. | 12 | Normal | 8.865 | 7.736 | 33 | 29 |
| Patient A. | 10 | Normal | 5.269 | 7.534 | 34 | 27 |
| Patient I. | 15 | Normal | 9.507 | 10.465 | 32 | 28 |
| Patient P. | 17 | Normal | 7.877 | 6.308 | 57 | 34 |
| Patient S. | 17 | Normal | 9.075 | 8.167 | 71 | 42 |
| Patient A. | 15 | Normal | 7.17 | 6.758 | 43 | 26 |
| Patient A. V. | 15 | Acute rhinitis | 7.814 | 6.716 | 36 | 15 |
| Patient A. R. | 14 | Chronic rhinitis | 0 | 0 | 23 | 12 |
| Patient A. S. | 14 | Acute rhinitis | 6.985 | 6.829 | 19 | 14 |
| Patient A. A. | 7 | Acute rhinitis | 8.302 | 7.1 | 26 | 14 |
| Average values | | | 7.0864 | 6.7613 | 37.4 | 24.1 |

**Table 18. Results for the test composition, Composition 6 (Tartarus stibiatus (alcoholic tincture), C12 dilution + Hepar sulphur (alcoholic tincture), C6 dilution + Apis mellifica (alcoholic solution), C30 dilution + Eupharsia officinalis (alcoholic tincture), DC 12 dilution + Allium cepa (alcoholic tincture), C6 dilution + Arnica montana (alcoholic tincture), C3 dilution)**

| **Name** | **Age** | **Abnormal or normal** | **Frequency 1, Hz** | **Frequency 2, Hz** | **Time 1, min** | **Time 2, min** |
|---|---|---|---|---|---|---|
| Patient B. | 9 | Acute rhinitis | 6.52 | 7.457 | 28 | 10 |
| Patient B.L . | 11 | Acute rhinitis | 6.085 | 7.298 | 31 | 20 |
| Patient B. R. | 17 | Chronic rhinitis | 8.873 | 7.738 | 41 | 29 |
| Patient D. | 6 | Acute rhinitis | 7.676 | 6.7 | 26 | 13 |
| Patient D. B. | 14 | Acute rhinitis | 9.38 | 8.945 | 79 | 26 |
| Patient D. A. | 8 | Acute rhinitis | 9.74 | 7.101 | 18 | 13 |
| Patient K. | 9 | Acute rhinitis | 7.553 | 8.663 | 18 | 28 |
| Patient K.L . | 11 | Acute rhinitis | 5.163 | 8.028 | 33 | 29 |
| Patient K. M. | 12 | Acute rhinitis | 7.812 | 10.04 | 44 | 27 |
| Patient K. N. | 8 | Acute rhinitis | 0 | 0 | 24 | 5 |
| Average values | | | 6.8802 | 7.197 | 34.2 | 20 |

**Table 19. Results for the test composition, Composition 7 (Tartarus stibiatus (alcoholic tincture), C6 dilution + Heparsulphur (alcoholic tincture), C12 dilution + Apis mellifica (alcoholic solution), C6 dilution + Eupharsia officinalis (alcoholic tincture), C30 dilution + Allium cepa (alcoholic tincture), C12 dilution + Arnica montana (alcoholic tincture), C6 dilution)**

| **Name** | **Age** | **Abnormal or normal** | **Frequency 1, Hz** | **Frequency 2, Hz** | **Time 1, min** | **Time 2, min** |
|---|---|---|---|---|---|---|
| Patient K. A. | 8 | Acute rhinitis | 8.801 | 5.466 | 24 | 23 |
| Patient K. B. | 14 | Acute rhinitis | 8.808 | 7.321 | 28 | 20 |
| Patient L. | 16 | Chronic rhinitis | 0 | 0 | 16 | 9 |
| Patient M. | 8 | Chronic rhinitis | 8.601 | 7.834 | 37 | 20 |
| Patient M. A. | 15 | Chronic rhinitis | 9.894 | 8.043 | 54 | 56 |
| Patient M. N. | 14 | Chronic rhinitis | 9.693 | 10.082 | 91 | 26 |
| Patient M. M. | 17 | Chronic rhinitis | 0 | 0 | 13 | 9 |
| Patient M. Z. | 17 | Acute rhinitis | 8.315 | 7.464 | 52 | 38 |
| Patient N. | 9 | Acute rhinitis | 8.205 | 9.72 | 18 | 17 |
| Patient N. O. | 4 | Acute rhinitis | 8.274 | 9.674 | 47 | 27 |
| Average values | | | 7.0591 | 6.5604 | 38 | 24.5 |

**Table 20. Results for the test composition, Composition 8 (Tartarus stibiatus (alcoholic tincture), C12 dilution + Hepar sulphur (alcoholic tincture), D6 dilution + Apis mellifica (alcoholic solution), C6 dilution+ Eupharsia officinalis (alcoholic tincture), D6 dilution + Allium cepa (alcoholic tincture), C12 dilution + Arnica montana (alcoholic tincture), C30 dilution)**

| **Name** | **Age** | **Abnormal or normal** | **Frequency 1, Hz** | **Frequency 2, Hz** | **Time 1, min** | **Time 2, min** |
|---|---|---|---|---|---|---|
| Patient O. | 8 | Acute rhinitis | 8.801 | 5.466 | 24 | 23 |
| Patient P. | 14 | Acute rhinitis | 8.808 | 7.321 | 28 | 20 |
| Patient P. O. | 16 | Acute rhinitis | 0 | 0 | 16 | 9 |
| Patient R. | 8 | Acute rhinitis | 8.601 | 7.834 | 37 | 20 |
| Patient S. A. | 15 | Acute rhinitis | 9.894 | 8.043 | 54 | 56 |
| Patient S. B. | 14 | Chronic rhinitis | 9.693 | 10.082 | 91 | 26 |
| Patient S. C. | 17 | Acute rhinitis | 0 | 0 | 13 | 9 |
| Patient S. N. | 17 | Acute rhinitis | 8.315 | 7.464 | 52 | 38 |
| Patient S. M. | 9 | Chronic rhinitis | 8.205 | 9.72 | 18 | 17 |
| Patient S. E. | 4 | Chronic rhinitis | 8.274 | 9.674 | 47 | 27 |
| Average values | | | 7.0591 | 6.5604 | 38 | 24.5 |

**Table 21. Results for the test composition, Composition 9 (Tartarus stibiatus (alcoholic tincture), C3 dilution + Heparsulphur (alcoholic tincture), C30 dilution + Apis mellifica (alcoholic solution), C6 dilution + Eupharsia officinalis (alcoholic tincture), C30 dilution + Allium cepa (alcoholic tincture), C12 dilution + Arnica montana (alcoholic tincture), D6 dilution)**

| **Name** | **Age** | **Abnormal or normal** | **Frequency 1, Hz** | **Frequency 2, Hz** | **Time 1, min** | **Time 2, min** |
|---|---|---|---|---|---|---|
| Patient S. | 16 | Acute rhinitis | 8.628 | 9.811 | 20 | 18 |
| Patient F. | 14 | Acute rhinitis | 8.045 | 8.097 | 39 | 32 |
| Patient E. | 12 | Chronic rhinitis | 0 | 0 | 2 | 2 |
| Patient J. | 9 | Chronic rhinitis | 7.98 | 6.394 | 25 | 16 |
| Patient J.A . | 12 | Chronic rhinitis | 6.447 | 7.852 | 30 | 44 |
| Patient N. | 13 | Chronic rhinitis | 5.71 | 7.037 | 31 | 44 |
| Patient M. | 13 | Acute rhinitis | 0 | 0 | 12 | 8 |
| Patient M.I. | 13 | Acute rhinitis | 0 | 0 | 4 | 3 |
| Patient Z. | 17 | Acute rhinitis | 0 | 0 | 10 | 12 |
| Patient Z. A. | 14 | Chronic rhinitis | 7.755 | 0 | 55 | 18 |
| Average values | | | 4.4565 | 3.9191 | 22.8 | 19.7 |

**Table 22. Results for the test composition, Composition 10 (Tartarus stibiatus (alcoholic tincture), C30 dilution + Hepar sulphur (alcoholic tincture), C30 dilution + Apis mellifica (alcoholic solution), C30 dilution + Eupharsia officinalis (alcoholic tincture), C30 dilution + Allium cepa (alcoholic tincture), C30 dilution + Arnica montana (alcoholic tincture), C30 dilution)**

| **Name** | **Age** | **Abnormal or normal** | **Frequency 1, Hz** | **Frequency 2, Hz** | **Time 1, min** | **Time 2, min** |
|---|---|---|---|---|---|---|
| Patient A. | 13 | Acute rhinitis | 6.685 | 9.756 | 45 | 29 |
| Patient O. | 17 | Chronic rhinitis | 9.049 | 8.894 | 26 | 23 |
| Patient P. | 14 | Chronic rhinitis | 8.449 | 9.36 | 35 | 32 |
| Patient R. | 17 | Acute rhinitis | 8.166 | 7.341 | 22 | 12 |
| Patient S. | 14 | Acute rhinitis | 9.528 | 10.14 | 20 | 12 |
| Patient S. A. | 11 | Acute rhinitis | 0 | 0 | 21 | 15 |
| Patient T. | 13 | Acute rhinitis | 9.079 | 7.631 | 21 | 12 |
| Patient T. A. | 17 | Chronic rhinitis | 8.679 | 9.535 | 26 | 12 |
| Patient F. | 15 | Chronic rhinitis | 6.272 | 4.991 | 45 | 33 |
| Patient C. | 17 | Acute rhinitis | 7.14 | 6.317 | 46 | 33 |
| Average values | | | 7.3047 | 7.3965 | 30.7 | 21.3 |

The study conducted by the authors of the present invention unexpectedly showed that, in comparison with the closest analogue, the composition of the invention is more effective, synergistically improves function of mucociliary clearance, slows down the respiratory tract ciliated epithelium cytolysis, and increases the lifespan of the respiratory tract ciliated epithelium, both in conditionally healthy people and in patients with acute and chronic rhinosinusitis in different age groups (both in children and adults) in invitro studies.

The study conducted by the authors of the present invention showed the following results:
1. The composition of the invention, in comparison with a saline, does not cause any cyto- and ciliary toxicity in all study groups of patients (i.e., it does not inhibit the ciliated epithelium action and does not lead to cytolysis (cell death)).
2. A slight fluctuation in the values of the ciliated epithelium cilia frequency beating is revealed when using the composition according to the invention and the saline solution. This may be mainly caused by the sensitivity of the ciliary apparatus functional activity to temperature factors and other inconsistencies during the study.
3. Increased average cell survival time in vitro is revealed when using the composition within the present invention (by 35.6% in the group of patients with chronic rhinitis, by 41.4% in the group of patients with acute rhinitis, by 61.3% in the relatively healthy group).
4. The best results in terms of cell survival in vitro were observed in the relatively healthy group. Thus, a dependence on the initial state of the cells was revealed (probably associated with the state and permeability of the cell membrane; the latter is disturbed in chronic and acute processes in the respiratory tract mucous membrane).

In sinusitis, it is important to address all stages of the disease at the same time, preventing the spread of infection, inflammation, and mucus congestion in the respiratory tract, since all three of these factors in the development of the disease are interconnected. A distinctive characteristic of the composition of the invention is the improvement of action of the upper and lower respiratory tract ciliated epithelium and the stimulation evacuation of the mucous membrane secretion. The amount of protective enzymes increases, and the local immunity of the upper and lower respiratory tract mucous membrane improves. Furthermore, due to its mucolytic features, the composition of the invention promotes cleansing of upper respiratory tract (nasal cavity, nasopharynx, oropharynx, and paranasal sinuses). Therefore, congestion in the sinuses and nasopharynx and bacterial activity and inflammation in the paranasal sinuses is reduced. Another feature of the treatment agent of the invention is its potential use after surgery and injuries. The good reparative characteristics of the composition of the invention make it possible to quickly heal wound beds of the mucous membrane and restore the ciliated epithelium due to its protective effect.

Based on the conducted study, the following conclusion can be made about the composition of the invention:
- it is effective in acute and exacerbated chronic inflammatory diseases of the upper respiratory tract selected from the group consisting of acute sinusitis (including maxillary frontal, and ethmoidal sinusitis, sphenoidal sinusitis, and polysinusitis), rhinitis, rhinosinusitis, nasopharyngitis, adenoiditis;
- it is effective for preventing inflammatory diseases of the mucous membrane the nasal cavity, nasopharynx, and paranasal sinuses, including improving the sense of smell by using the composition topically intranasally or by irrigation or washing the nasal cavity and/or paranasal sinuses in sufficient quantities for 1-2 months.
- it can be used as a primary or additional agent in combined therapy and for the prevention of acute respiratory viral infections, acute respiratory infections, influenza, and other colds.
- another aspect of the invention is the use of the composition as a primary or additional agent in combined therapy after injuries and/or surgeries of the mucous membrane on the nasal cavity, paranasal sinuses, nasopharynx.
- yet another aspect of the invention is the use of the composition after cosmetic, plastic or maxillofacial, and dental surgeries on the nasal cavity and paranasal sinuses.
- yet another aspect of use is moisturizing the mucous membrane if mucous membrane is dryness of any etiology, in particular in smokers and passengers after long flights on airplanes, as well as for hypertrophic and atrophic rhinitis.
- yet another aspect of the invention is the use of the composition as a primary or additional agent in the complex therapy of allergic rhinitis, atrophic rhinitis, vasomotor rhinitis, and as an additional agent for exudative otitis media and salpingootitis.

Thus, the claimed composition may reduce the amount of antibacterial, antiviral, and hormonal agents used and improve the quality of life of patients with acute and chronic diseases of the nasal cavity, nasopharynx, and paranasal sinuses.

### References:

1. Fokkens WJ, Lund VJ et al. EPOS 2012 // Rhinology 2012 Vol.50 Suppl. 23. No. 3, p. 1-298.
2. Fokkens W, Lund V, Mullol J. European position paper on rhinosinusitis and nasal polyps 2007. // Rhinol Suppl. 2007(20): 1-136.
3. Desrosiers M, Evans GA, Keith PK, Wright ED, Kaplan A, Bouchard J, et al. Canadian clinical practice guidelines for acute and chronic rhinosinusitis. // Journal of otolaryngology-head & neck surgery = Le Journal d'oto- rhino-laryngologie et de chirurgie cervico-faciale. 2011 May; 40 Suppl 2:S99-193.
4. Wang DY, Wardani RS, Singh K, Thanaviratananich S, Vicente G, Xu G, et al. A survey on the management of acute rhinosinusitis among Asian physicians. // Rhinology. 2011 Sep; 49(3):264-71.
5. Chan Y, Kuhn FA. An update on the classifications, diagnosis, and treatment of rhinosinusitis. // Current opinion in otolaryngology & head and neck surgery. 2009 Jun;17(3):204-8.
6. Marple BF, Brunton S, Ferguson BJ. Acute bacterial rhinosinusitis: A review of U.S. treatment guidelines. // Otolaryngology-Head & Neck Surgery. [Review]. 2006;135(3):341-8.
7. Van Gageldonk-Lafeber AB, Heijnen ML, Bartelds Al, Peters MF, van der Plas SM, Wilbrink B. A case-control study of acute respiratory tract infection in general practice patients in The Netherlands. // Clin Infect Dis. 2005 Aug 15; 41(4):490-7. Epub 2005 Jul 15; PMID: 16028157.
8. Bachert C, Hormann K, Mosges R, Rasp G, Riechelmann H, Muller R, et al. An update on the diagnosis and treatment of sinusitis and nasal polyposis. // Allergy. 2003 Mar; 58(3): 176-91.
9. Uijen JH, Bindels PJ, Schellevis FG, van der Wouden JC. ENT problems in Dutch children: trends in incidence rates, antibiotic prescribing and referrals 2002-2008. // Scand J Prim Health Care. 2011 Jun; 29(2):75-9.
10. Cherry DK, Woodwell DA, Rechtsteiner EA. National Ambulatory Medical Care Survey: 2005 summary. // Adv Data. 2007 Jun 29(387): 1-39.
11. Bhattacharyya N. Contemporary assessment of the disease burden of sinusitis. The economic burden and symptom manifestations of chronic rhinosinusitis. // Am J Rhinol Allergy. 2009; 23(4):392-5.
12. Bhutta MF, Al-Shaikh S, Latif M, Lee R, Uraiby J. Nasal polyps do not contain olfactory structures. // Rhinology. 2011 Jun; 49(2): 185-9.
13. Louie JK, Hacker JK, Gonzales R, Mark J, Maselli JH, Yagi S, et al. Characterization of viral agents causing acute respiratory infection in a San Francisco University Medical Center Clinic during the influenza season. // Clinical infectious diseases: an official publication of the Infectious Diseases Society of America. 2005 Sep 15; 41(6):822-8.
14. Varonen H, Rautakorpi UM, Huikko S, Honkanen PO, Klaukka T, Laippala P, et al. Management of acute maxillary sinusitis in Finnish primary care. Results from the nationwide MIKSTRA study. // Scand J Prim Health Care. 2004 Jun; 22(2): 122-7.
15. Wang DY, Wardani RS, Singh K, Thanaviratananich S, Vicente G, Xu G, et al. A survey on the management of acute rhinosinusitis among Asian physicians. // Rhinology. 2011 Sep; 49(3):264-71.
16. Gijsen R., Poos M. National Kompas 2003 [cited; Available from: Gwaltney JM, Jr Acute community-acquired sinusitis. Clin Infect Dis [1996; 23(6): 1209-23; quiz.24-5].
17. Felmingham D1, Feldman C, Hryniewicz W, Klugman K, Kohno S, Low DE, Mendes C, Rodloff AC. Surveillance of resistance in bacteria causing community-acquired respiratory tract infections. // Clin Microbiol Infect. 2002;8 Suppl 2:12-42.
18. Hoban D, Felmingham D. The PROTEKT surveillance study: antimicrobial susceptibility of Haemophilus influenzae and Moraxella catarrhalis from community-acquired respiratory tract infections. // J Antimicrob Chemother. 2002 Sep; 50 Suppl S 1:49-59. PMID: 12239228.
19. Benninger MS. Acute bacterial rhinosinusitis and otitis media: changes in pathogenicity following widespread use of pneumococcal conjugate vaccine. Otolaryngology-head and neck surgery: official journal of American Academy of Otolaryngology-Head and Neck Surgery. 2008 Mar;138(3):274-8.
20. Bachert C, Schapowal A, Funk P, Kieser M. Treatment of acute rhinosinusitis with the preparation from Pelargonium sidoides EPs 7630: a randomized, double-blind, placebo-controlled trial. // Rhinology. 2009 Mar;47(1):51-8.
21. Chen Y, Dales R, Lin M. The epidemiology of chronic rhinosinusitis in Canadians. // The Laryngoscope. 2003 Jul; 113(7): 1199-205.
22. Friedman WH. Surgery for chronic hyperplastic rhinosinusitis. // Laryngoscope. 1975 Dec;85(12pt 1): 1999-2011; PMID:1202305.
23. Maresh MM, Wahbum A. Paranasal sinuses from birth to late adolescence. Clinical and roentgengraphic evidence of infection. // Am.J.Dis Child 1940 (60): 841-84.
24. Bagatsch K DK, Paethenheimer F, Ritter B. Morbidates analyse der unspezifischinfekthedingten acute Erkrankungen der Respirationtraktes und der Mittelohrraume des Kinderesaltems in einem Ballunsgebiet mit modernen Wohnbedingungen. // HNO Praxis. 1980(5): 1-8.
25. Gordts F, Clement PA, Destryker A, Desprechins B, Kaufman L. Prevalence of sinusitis signs on MRI in a non-ENT paediatric population. // Rhinology. 1997 Dec; 35(4): 154-7; PMID:9532633.
26. van der Veken PJ1, Clement PA, Buisseret T, Desprechins B, Kaufman L, Derde MP. CT-scan study of the incidence of sinus involvement and nasal anatomic variations in 196 children. // Rhinology. 1990 Sep;28(3): 177-84.
27. Ray NF, Baraniuk JN, Thamer M, Rinehart CS, Gergen PJ, Kaliner M, Josephs S, Pung YH. Healthcare expenditures for sinusitis in 1996: contributions of asthma, rhinitis, and other airway disorders. //J Allergy Clin Immunol. 1999 Mar; 103(3 Pt I):408-14. PMID: 10069873.
28. Stankiewicz JA, Chow JM. Cost analysis in the diagnosis of chronic rhinosinusitis. // Am J Rhinol. 2003 May-Jun; 17(3): 139-42. PMID: 12862401.
29. Franzese CB, Stringer SP. Economic analysis of the use of limited coronal computed tomography scans in the management of sinusitis. // Am J Rhinol. 2004 Sep-Oct;18(5):329-34; PMID: 15586806.
30. Blackwell DL1, Collins JG, Coles R. Summary health statistics for U.S. adults: National Health Interview Survey, 1997. // Vital Health Stat 10. 2002 May; (205): 1-109.
31. Murphy MP1, Fishman P, Short SO, Sullivan SD, Yueh B, Weymuller EA Jr. Health care utilization and cost among adults with chronic rhinosinusitis enrolled in a health maintenance organization. // Otolaryngol Head Neck Surg. 2002 Nov; 127(5):367-76.
32. Van Agthoven M, Uyl-de Groot CA, Fokkens WJ, van de Merwe JP, Busschbach JJ. Cost analysis of regular and filgrastim treatment in patients with refractory chronic rhinosinusitis. // Rhinology. 2002 Jun; 40(2):69-74. PMID: 12091996.
33. Goetzel RZ, Hawkins K, Ozminkowski RJ, Wang S. The health and productivity cost burden of the top 10 physical and mental health conditions affecting six large U.S. employers in 1999. // J Occup Environ Med. 2003 Jan; 45(1):5-14. PMID: 12553174.
34. Bhattacharyya NI The economic burden and symptom manifestation of chronic rhinosinusitis. // Am. J Rhinol. 2003; 17(I):27-32.
35. Gliklich RE, Metson R. Economic implications of chronic sinusitis. // Otolaryngol Head Neck Surg. 1998 Mar; 118(3 Pt 1):344-9. PMID: 9527115.
36. Wasserfallen JB1, Livio F, Zanetti G. Acute rhinosinusitis: a pharmacoeconomic review of antibacterial use. // Pharmacoeconomics. 2004;22(13):829-37.

## Claims

1. A pharmaceutical composition for treating or preventing acute and chronic inflammatory, allergic, post-traumatic, atrophic, and hypertrophic diseases of the upper and lower respiratory tract **characterized in that** the composition comprises alcoholic tinctures of *Tartarus stibiatus, Hepar sulfur, Euphrasia officinalis, Allium sulfur,* and *Arnica montana* and an alcoholic solution of *Apis mellifica* combined with a physiologically acceptable carrier.

2. The pharmaceutical composition according to Claim 1 **characterized in that** a volume ratio of the alcoholic tinctures of Tartarus stibiatus, Hepar sulfur, Euphrasia officinalis, Allium sulfur, and Arnica montana and the alcoholic solution of Apis mellifica on the one part and a physiologically acceptable liquid carrier on the other part is 0.5 ml : 100 ml to 15 ml : 100 ml.

3. The pharmaceutical composition according to any one of Claims 1 or 2 **characterized in that** the physiologically acceptable liquid carrier is a 0.9% sodium chloride, water, or a gelling or other base.

4. The pharmaceutical composition according to Claim 4 **characterized in that** the gelling base is selected from polyacrylic acid, copolymers of acrylic and methacrylic acids, cellulose and cellulose derivatives, polyvinyl resins, alginic acid and its pharmaceutically acceptable salts, or combinations thereof.

5. The pharmaceutical composition according to any one of Claims 1 or 2 **characterized in that** it further comprises excipients selected from stabilizers, pH regulators, thickeners, and preservatives.

6. The pharmaceutical composition according to any one of Claims 1 or 2 **characterized in that** alcoholic tinctures of Euphrasia officinalis, Allium sulphate, Arnica montana are alcoholic tinctures or homeopathic alcoholic tinctures in a dilution, independently in each case, ranging from D3 to C30, alcoholic tinctures of Tartarus stibiatus and Hepar sulfur are homeopathic alcoholic tinctures with dilutions, independently in each case, ranging from D3 to C30, and the alcoholic solution of Apis mellifica is a homeopathic alcoholic solution with a D3 to C30 dilution.

7. Use of the pharmaceutical composition according to any one of Claims 1-6 for treating or preventing acute and chronic inflammatory, allergic, post-traumatic, atrophic, and hypertrophic diseases of the upper and lower respiratory tract.
